# EUROPEAN PATENT APPLICATION

(11) **EP 3 913 063 A1**
(43) Date of publication of application: **24.11.2021**
(21) Application number: 20175843.0
(22) Date of filing: 20.05.2020
(51) Int. Cl.: C12P 17/06, C12N 9/02

(54) **NON-NATURALLY OCCURRING MICROORGANISM FOR THE BIOSYNTHETIC PRODUCTION OF BAICALEIN**

(71) Applicant: Synbionik GmbH, 60314 Frankfurt am Main (DE)
(72) Inventor: SCHMITT, Patrick, 55122 Mainz (DE); NEUKRANZ, Yannika, 60598 Frankfurt am Main (DE); MACIEJKO, Jakob, 65239 Hochheim am Main (DE); TABOADA, Andrea, 60439 Frankfurt am Main (DE)
(74) Representative: Kilger, Ute

(57) **Abstract**

The invention describes a non-natural occurring microorganism used for the biosynthetic production of baicalein from the precursor molecule chrysin. This is enabled, in the microorganism, of cytochrome P450 CYP82D1.2 (protein ID: ASW21051.1) from *Scutellaria baicalensis* and a cytochrome P450 reductase to ensure proper function of the first. This invention proposes an alternative and scalable production method to the mainstream method currently used, plant extraction. So, that baicalein can be introduced into the pharmaceutical and medical market as a principal or supplementary medication to patients.

## Description

### Background

Baicalein (5,6,7-trihydroxyflavone), the aglycone of baicalin, is a type of flavonoid, which was originally isolated from the roots of *Scutellaria baicalensis* and *Scutellaria lateriflora.* Also, It is reported in *Oroxylum indicum* (Indian trumpet flower) and Thyme (Matsumoto et al. 2008).

Some of the properties of baicalein include being a positive allosteric modulator (a group of substances that bind to a receptor to change that receptor's response to stimulus) of the benzodiazepine site and/or a non-benzodiazepine site of the GABA receptor (the major inhibitory neurotransmitter in the central nervous system) (Wang et al. 2002) (Hui et al. 2000) (Liao et al. 1998) (Edwin et al. 2004). Additionally, baicalein is an antagonist of the estrogen receptor, also known as antiestrogen (Schwartz et al. 2008). This flavonoid, baicalein, has shown to inhibit some lipoxygenases (a family of iron-containing enzymes most of which catalyze the deoxygenation of polyunsaturated fatty acids in lipids) (Deschamps et al. 2006) and act as an anti-inflammatory agent *(* Hsieh et al. 2007). Baicalein has antiproliferative properties via inhibition of TRPC1 channel on pulmonary artery smooth muscle cells (Lin et al. 2011). Research performed in animals suggest that baicalein could have antidepressant effects (Xiong et al. 2011). Moreover, baicalein presents anxiolytic effects (inhibition of anxiety) in mice without the need of sedation or myorelaxation (de Carvalho et al. 2011) (Wang et al. 2002) (Liao et al. 1998). It has been shown that baicalein is an inhibitor of CYP2C9, an enzyme of the cytochrome P450 system (Si D et al. 2009). As well, baicalein has been shown in vitro to inhibit the biofilm formation and the quorum sensing system of the microorganism *Staphylococcus aureus* (Chen et al. 2016). Overall, baicalein has acquired attention from the medical, biotechnological and pharmaceutical industry due to its properties and broad range of use. This makes baicalein a potential natural candidate to be part of the mainstream Western medicine either for use as treatment and/or to support other medicaments. Consequently, this will broaden the possibilities of treatment for more patients and will offer a more natural option.

However, due to the natural origin of baicalein (primarily obtained from extraction from the roots of *Scutellaria baicalensis* and *Scutellaria lateriflora)* the current available method of supply is not able to meet the rising demand. This means that, through natural processes (plant extraction) it would neither be suitable nor sustainable to provide supply to a large population. Nevertheless, the lack of good manufacturing practices (G.M.P's.) decreases the possibilities of this natural compound, baicalein, of becoming a candidate for mainstream pharmacological products and for being incorporated in current medical practices. In addition, if baicalein becomes a substance for mainstream it will be required a method able to sustain a constant production of baicalein in order to provide supply to a large population of patients either for research, development, treatment or treatment support. If the method of supply is only limited to the extraction of baicalein from plants, it would not be enough to reach the quantity nor quality requirements. Plus, the environmental impact that this practice can produce (due to the water necessities and soil manipulation with pesticides and other chemicals) will be counter beneficial long-term for the stability of the environment overall. Therefore, the solution to this problem should be a production method of baicalein with a high yield, while meeting the sanitation and manufacturing standards and have the characteristic in order to achieve scalability. For that reason, alternative methods to plant extraction have been proposed.

CN103160549A describes a method for preparing, separating and purifying baicalein from endogenous enzymes. The main features described are: using traditional Chinese medicine baicalein as raw material, using original endogenous enzyme-induced biotransformation technology, a series of highly efficient transformation, extraction, separation and purification techniques such as negative pressure cavitation extraction technology, liquid-liquid extraction technology, normal-phase silica gel medium and low-pressure preparative liquid chromatography technology, and low-temperature crystallization and recrystallization technology have obtained *Scutellaria baicalensis* with a purity greater than 98%. However, this proposed method does not propose a solution to the presented problem mainly because it requires starting material baicalin, which would need to be extracted from plans. Therefore, increasing the cost of production and environmental impact. Additionally, multiple extraction methods are required increasing the number of steps required. Consequently, raising the cost of production and increasing the price of the product could cause a possible decrease of demand. Therefore, decreasing the reasons to achieve the introduction of this natural compound, baicalein.

WO2005045051A1 describes a process for producing baicalein comprising a method for de-glycosylation of baicalin to produce baicalein by treating the baicalin with an enzyme having beta-glucuronidase activity under conditions such that baicalin is converted to baicalein with at a conversion rate of at least 70%. However, this proposed method does not present a solution to the presented problem mainly because baicalin, the glucuronide of baicalein, is required as the raw starting material. This means that plan extraction to produce baicalein is still required. Consequently, making this process have a high in cost and environmental impact. Therefore, not resolving the problem described before. Additionally, this method only has a 70% of conversion rate. Therefore, there is a 30% of lost in production per batch. Consequently, for every batch produced of baicalein, the manufacturing company will not be able to reach maximum profit from its production. Therefore, this process will not be appealing to large industries such as the pharmaceutical industry, which seek optimal processes by using low cost raw materials and having more than 90% formation of a product per batch.

CN105238826A describes a method for preparing baicalein through the beta-glucosidase. The heat-resisting and sugar-resisting beta-glucosidase is immobilized to hydrolyze the baicalin in a buffer solution, and the baicalein is obtained. According to the method, after the baicalein is extracted through ethyl acetate, recrystallization can be carried out through organic solvents, the high purity baicalein can be obtained, and the purity is higher than 98%. However, this method does not resolve the proposed problem because further research would be needed in order to show that no residues of the solvents are in the final product. Additionally, further testing is required to show that no harm to patients could be done by any possible residue of the solvents in the product. Due to this additional testing required, the cost of developing this method will increase. Moreover, this method does would not be efficient to be scalable. Because for every batch of production intended for large supplies, big quantities of solvents will be required. Therefore, making the cost of production become higher. Consequently, each batch of product will be very costly and raising the price of the product making this method not appealing to the pharmaceutical industry.

US7771758B2 describes a composition comprising an extract of *Scutellaria lateriflora* including baicalein, scutellarin, dihydrobaicalin, ikonnikoswide I, lateriflorin, baicalein, lateriflorein and wogonin. Said extract is produced by subjecting dried *Scutellaria lateriflora* plant material with a solvent. More specifically, a solvent consisting of water at a temperature of about 65° C. to about 95° C.. However, this is does not solve the stated problem because the raw material is required to obtain baicalein. Additionally, this is the least efficient method since in each extraction multiple products are produced decreasing the final concentration of baicalein. Therefore, this method will have a high environmental impact if scalable. Consequently, not being appealing to the pharmaceutical industry because it will cost too much to supply a high demand. Also, making less expensive options more suitable for be used for treatment.

### Brief Description of the Invention

The invention describes a non-naturally occurring microorganism used for the biosynthetic production of baicalein from the precursor molecule chrysin. This is enabled by the overexpression, in the microorganism, of cytochrome P450 CYP82D1.2 (protein ID: ASW21051.1) from *Scutellaria baicalensis* in order to catalyze the reaction from chrysin to baicalein and it is selected from P450 CYP82D1.2 (identified as CYPD1.2_whole and by SEQ ID_NO 1) (protein ID: ASW21051.1), CYPD1.2_TR (identified as SEQ ID_NO 2) and CYPD1.2_TR_SOL (identified as SEQ ID_NO 3). Additionally, a reductase sequence is needed in order to ensure the functionality of cytochrome P450 CYPD1.2. Therefore, a cytochrome P450 reductase sequence from *Arabidopsis thaliana,* which is selected from P450 reductase AtATR2 (Q9SUM3) (identified as AtATR2), AtATR2_TR (identified as SEQ ID_NO 5), cytochrome P450 reductase CPR1 (Q9SB48.1) (identified as CPR1 and SEQ ID_NO 6) and CPR1_TR (identified as SEQ ID_NO 7). The selected genes are used from the plant *Scutellaria baicalensis,* which naturally produces baicalein.

This invention can be placed in the industry of biotechnology since it involves the use of genetic engineering to modify the non-naturally occurring microorganism. Plus, this invention seeks to support the pharmacological industry by generating an efficient and scalable production of baicalein using microorganisms as producers. This means that the production of baicalein can be continuous, non-stopped and with minimal human intervention in the process. In addition, this method does not require the use of hard chemicals, which makes this method sustainable having a low environmental impact. Moreover, since the non-occurring microorganism will only require chrysin as a raw material, scalable production will be efficient and making the production have a low-cost.

### Detailed Description of the Invention

Subject matter of the present application is a non-natural occurring microorganism which is able to biosynthetically produce baicalein (5,6,7-trihydroxyflavone), wherein said microorganism has been modified to comprise an enzymatic pathway for the biosynthetic production of baicalein.

One embodiment of the present application relates to a non-natural occurring microorganism which is able to biosynthetically produce baicalein, wherein the precursor for the biosynthetic production of baicalein is chrysin.

One preferred embodiment of the present application relates to a non-natural occurring microorganism which is able to biosynthetically produce baicalein, wherein said microorganism is genetically modified.

Another embodiment of the present application relates to a non-natural occurring microorganism which is able to biosynthetically produce baicalein, wherein Chrysin is used as a substrate and is converted to baicalein.

Another embodiment of the present application relates to a non-natural occurring microorganism which is able to biosynthetically produce baicalein, wherein said enzymatic pathway comprises at least one cytochrome and at least one cytochrome reductase.

One specific embodiment of the present application relates to a non-natural occurring microorganism which is able to biosynthetically produce baicalein, wherein said microorganism is expressing cytochrome P450 CYP82D1.2 and cytochrome P450 reductase.

Another embodiment of the present application relates to a non-natural occurring microorganism which is able to biosynthetically produce baicalein, wherein said microorganism has been modified to comprise cytochrome P450 and cytochrome P450 reductase.

Another preferred embodiment of the present application relates to non-natural occurring microorganism which is able to biosynthetically produce baicalein, wherein said microorganism has been modified to comprise cytochrome P450 CYP82D1.2 and cytochrome P450 reductase.

Another specific embodiment of the present application relates to a non-natural occurring microorganism which is able to biosynthetically produce baicalein, wherein cytochrome P450 CYP82D1.2 is overexpressed in said microorganism for the biosynthetic production of baicalein.

One embodiment of the present application relates to a non-natural occurring microorganism, wherein the cytochrome P450 CYP82D1.2 is selected from SEQ ID_NO 1 or a protein sequence having at least 70% identity thereof, SEQ ID_NO 2 or a protein sequence having at least 70% identity thereof, and SEQ ID_NO 3 or a protein sequence having at least 70% identity thereof, and the cytochrome P450 reductase is selected from SEQ ID_NO 4 or a protein sequence having at least 70% identity thereof, SEQ ID_NO 5 or a protein sequence having at least 70% identity thereof, SEQ ID_NO 6 or a protein sequence having at least 70% identity thereof, and SEQ ID_NO 7 or a protein sequence having at least 70% identity thereof.

Another embodiment of the present application relates to a non-natural occurring microorganism, wherein the cytochrome P450 CYP82D1.2 is selected from SEQ ID_NO 1 or a protein sequence having at least 80% identity thereof, SEQ ID_NO 2 or a protein sequence having at least 80% identity thereof, and SEQ ID_NO 3 or a protein sequence having at least 80% identity thereof, and the cytochrome P450 reductase is selected from SEQ ID_NO 4 or a protein sequence having at least 80% identity thereof, SEQ ID_NO 5 or a protein sequence having at least 80% identity thereof, SEQ ID_NO 6 or a protein sequence having at least 80% identity thereof, and SEQ ID_NO 7 or a protein sequence having at least 80% identity thereof.

Another embodiment of the present application relates to a non-natural occurring microorganism, wherein the cytochrome P450 CYP82D1.2 is selected from SEQ ID_NO 1 or a protein sequence having at least 90% identity thereof, SEQ ID_NO 2 or a protein sequence having at least 90% identity thereof, and SEQ ID_NO 3 or a protein sequence having at least 90% identity thereof, and the cytochrome P450 reductase is selected from SEQ ID_NO 4 or a protein sequence having at least 90% identity thereof, SEQ ID_NO 5 or a protein sequence having at least 90 % identity thereof, SEQ ID_NO 6 or a protein sequence having at least 90% identity thereof, and SEQ ID_NO 7 or a protein sequence having at least 90% identity thereof.

Another embodiment of the present application relates to a non-natural occurring microorganism, wherein SEQ ID_NO 1, SEQ ID_NO 2, SEQ ID_NO 3, SEQ ID_NO 4, SEQ ID_NO 5, SEQ ID_NO 6 and SEQ ID_NO 7 have at least an 85% of identity to any of the herein claimed sequences SEQ ID NO 1-7.

Another embodiment of the present application relates to a non-natural occurring microorganism, wherein said microorganism is genetically modified with SEQ ID_NO 3 or a protein sequence having at least 70% identity thereof, and SEQ ID_NO 4 or a protein sequence having at least 70% identity thereof.

Another embodiment of the present application relates to a non-natural occurring microorganism, wherein said microorganism is genetically modified with SEQ ID_NO 3 or a protein sequence having at least 80% identity thereof, and SEQ ID_NO 4 or a protein sequence having at least 80% identity thereof.

Another specific embodiment of the present application relates to a non-natural occurring microorganism, wherein said microorganism is genetically modified with SEQ ID_NO 3 or a protein sequence having at least 90% identity thereof, and SEQ ID_NO 4 or a protein sequence having at least 90% identity thereof.

Another embodiment of the present application relates to a non-natural occurring microorganism, wherein said microorganism is genetically modified with SEQ ID_NO 3 or a protein sequence having at least 70% identity thereof, and SEQ ID_NO 6 or a protein sequence having at least 70% identity thereof.

Another embodiment of the present application relates to a non-natural occurring microorganism, wherein said microorganism is genetically modified with SEQ ID_NO 3 or a protein sequence having at least 80% identity thereof, and SEQ ID_NO 6 or a protein sequence having at least 80% identity thereof.

Another embodiment of the present application relates to a non-natural occurring microorganism, wherein said microorganism is genetically modified with SEQ ID_NO 3 or a protein sequence having at least 90% identity thereof, and SEQ ID_NO 6 or a protein sequence having at least 90% identity thereof.

One embodiment of the present application relates to a non-natural occurring microorganism, wherein said microorganism is a bacterium.

Another embodiment of the present application relates to a non-natural occurring microorganism, wherein said bacterium is from the *Escherichia* genus.

Another preferred embodiment of the present application relates to a non-natural occurring microorganism wherein said bacteria is selected between *Escherichia coli BLR (DE3), Escherichia coli Rosetta2 (DE3), Escherichia coli T7* and *Express Escherichia coli BL21 (DE3).*

Another embodiment of the present application relates to a non-natural occurring microorganism, wherein said bacterium is from the *Zymomonas* genus.

Another specific embodiment of the present application relates to a non-natural occurring microorganism, wherein said bacteria is *Zymomonas mobilis.*

One embodiment of the present application relates to a non-natural occurring microorganism, wherein said bacterium is from the *Clostridium* genus.

One preferred embodiment of the present application relates to a non-natural occurring microorganism, wherein said bacterium is from the *Clostridium autoethanogenum.*

Another embodiment of the present application relates to a non-natural occurring microorganism, wherein said microorganism is a eukaryote.

Another preferred embodiment of the present application relates to a non-natural occurring microorganism, wherein said eukaryote is a *Saccharomyces.*

Another specific embodiment of the present application relates to a non-natural occurring microorganism, wherein said eukaryote is *Saccharomyces Cerevisiae*

Subject-matter of the present application relates also to a method method to produce baicalein, comprising a non-natural occurring microorganism, wherein said microorganism is defined in the aforementioned embodiments.

Subject-matter of the present application relates also to a method to produce baicalein, comprising a non-natural occurring microorganism, wherein said microorganism is defined in the aforementioned embodiments, and in particular wherein said microorganism has been modified to comprise cytochrome P450 CYP82D1.2 and cytochrome P450 reductase.

Further subject-matter of the present application relates to a method to produce baicalein comprising a non-natural occurring microorganism, which has been modified to comprise cytochrome P450 CYP82D1.2 and cytochrome P450 reductase.

Subject-matter of the present application relates also to an use of a non-natural occurring microorganism as defined in the aforementioned embodiments for biosynthetic production of baicalein from the precursor molecule chrysin.

Further subject-matter of the present application relates to an use of a non-natural occurring microorganism as defined in the aforementioned embodiments for biosynthetic production of baicalein from the precursor molecule chrysin, and in particular wherein said microorganism has been modified to comprise cytochrome P450 CYP82D1.2 and cytochrome P450 reductase.

Subject-matter of the present application relates also to an use of a non-natural occurring microorganism for biosynthetic production of baicalein from the precursor molecule chrysin, wherein said microorganism has been modified to comprise cytochrome P450 CYP82D1.2 and cytochrome P450 reductase.

### Strain Selection to produce Baicalein

The microorganism is genetically modified to comprises exogenous genetic material coding for the enzymatic pathway of baicalein.

The microorganism selected for the genetic modification to produce baicalein must comprise or present the following characteristics:
1) There must be available genetic tools for the selected microorganism in order to incorporate genetic modifications. The microorganism must be able to undergo genetic modification via plasmid transformation or genome integration, deletion and modification.
2) The selected microorganism must have a scalable and simple downstream and upstream processing, while at the same time have a low cost to develop them.
3) The selected microorganism must be able to metabolize the desired product utilizing its natural transporter system.
4) The selected microorganism must be efficient in its metabolism in order to produce the desired amount of compound.

The microorganism is genetically modified in a way that it comprises exogenous genetic material coding for the enzymatic pathway of baicalein.

Exogenous genetic material refers to DNA originating outside the organism of concern or study.

In an embodiment the genetically modified microorganism is selected from the genus *Escherichia, Saccharomyces, Clostridium, Bacillus, Lactococcus, Zymomonas, Corynebacterium, Pichia, Candida, Hansenula, Trichoderma, Acetobacterium, Ralstonia, Cupravidor, Salmonella, Klebsiella, Paenibacillus, Pseudomonas, Lactobacillus, Rhodococcus, Enterococcus, Alkaligenes, Brevibacterium, Methylobacterium, Methylococcus, Methylomonas, Methylocystis* and *Methylosinus.*

In a preferred embodiment, the microorganism is selected from the group comprising of *Escherichia coli, Saccharomyces cerevisiae, Clostridium acetobutylicum, Clostridium beijerinckii, Clostridium saccharbutyricum, Clostridium saccharoperbutylacetonicum, Clostridium butyricum, Clostridium diolis, Clostridium kluyveri, Clostridium pasterianium, Clostridium novyi, Clostridium difficile, Clostridium thermocellum, Clostridium cellulolyticum, Clostridium cellulovorans, Clostridium phytofermentans, Lactococcus lactis, Bacillus subtilis, Bacillus licheniformis, Zymomonas mobilis, Klebsiella oxytoca, Klebsiella pneumonia, Corynebacterium glutamicum, Trichoderma reesei, Ralstonia eutropha, Cupriavidus necator, Pseudomonas putida, Lactobacillus plantarum* and *Methylob acterium extorquens.*

In another embodiment the genetically modified microorganism is selected from a group comprising a carboxydotrophic bacteria from the genus *Clostridium, Moorella, Oxobacter, Acetobacterium, Eubacterium* or *Butyribacterium.*

In a preferred embodiment the carboxydotrophic microorganism is selected from a group comprising *Clostridium Ijungdahlii, Clostridium carboxydivorans, Clostridium ragsdalei, Clostridium autoethanogenum, Moorella thermoacetica, Moorella thermoautotrophica, Oxobacter pfennigii, Acetobacterium woodi, Eubacterium limosum* and *Butyribacterium methylotrophicum.*

In a preferred embodiment the microorganism is *Escherichia coli BLR (DE3)* as it is used for the heterologous expression of unstable proteins or enzymes *(Goffin et. al. 2017).*

In a preferred embodiment the microorganism is *Escherichia coli Rosetta2 (DE3)* as it is used for the heterologous expression of eukaryotic proteins or enzymes (Kopanic et. al. 2013).

In a preferred embodiment the microorganism is *Escherichia coli T7 Express* as it is an organism of choice for the general heterologous expression and production of recombinant proteins or enzymes (Lobstein et. al. 2012).

In a preferred embodiment the microorganism is *Escherichia coli BL21 (DE3)* as it is an organism of choice for the heterologous expression and production of recombinant proteins or enzymes (Rosano et al 2014).

In a preferred embodiment the microorganism is *Zymomonas mobilis* ZM4 as it contains a number of desirable characteristics such as the Entner-Doudoroff pathway and as a consequence makes it a suitable microorganism for metabolic engineering and production of bio-products (e.g. baicalein) (He et al 2014).

In a preferred embodiment the microorganism is *Saccharomyces Cerevisiae* as it is a eukaryotic microorganism and has advantages for the heterologous expression of eukaryotic (plant) enzymes. The robust cultivation and genetic modification of the microorganism is advantageous for metabolic engineering and expression of heterologous enzymes (Borgne 2012).

In a preferred embodiment the microorganism is *Clostridium autoethanogenum* as it is an anaerobic microorganism. Therefore, it can utilize an alternative carbon source. The carbon source for the fermentation reaction is a gaseous substrate containing at least one of CO, CO2 and H₂. The substrate may be a waste gas obtained as a by-product of an industrial process (for example steel manufacturing, gasification of biomass, coal, animal wastes, production of ferroalloys and municipal solid waste (Sun et al. 2019)), or from another source such as from automobile exhaust fumes (Xu et al. 2017).

### Genetic Modification of the Microorganism

The non-naturally occurring microorganism comprises one or more genes, which enable the expression of enzymes to produce baicalein.

A) The enzymatic pathway in the non-naturally microorganism to produce baicalein from chrysin comprises a cytochrome P450 CYP82D1.2, which is selected from the following sequences:
In an embodiment, cytochrome P450 CYP82D1.2 (identified as CYPD1.2_whole) (protein ID: ASW21051.1) from *Scutellaria baicalensis* is used. The following sequence CYPD1.2_whole (identified as SEQ ID_NO 1) comprises the amino acid primary sequence:

MELSSVIYGAIALLSLFYCYLHFSKPKKSSLNAPPEAGGARFITGHLHLMDGRSASDK
LPHINLGLLADQHGPIFTIRLGVHRAVVVSSWELAKEIFTTHDTAVMARPRLIADDYLS
YDGASLGFSPYGPYWREIRKLVTTELYKLWEEKKDGSGRVLVDMKQWLGNLSLNLV
SRMVVGKRFYGGDDSETTKRWRGVMREFFQLIGQFIPGDGLPFLRWLDLGGFEKR
TRDTAYELDKIIAMWLAEYRKREYSGDDKEQCFMALMLSLVQANPTLQLHYDADTIIK
ATCQVLISAASDTTTVILIWVISLLLNNADVLKKVQEELDEQVGRERRVEESDISNLPY
LQAVVKETMRLYPPAPFAGVRAFSEDCTVGGYHIQKGTFLIVNLWKLHRDPRVWSD
DALEFKPQRFFDKKVEVKGQDFELMPFGGGRRMCPGSNLGMHMVHFVLANILQAF
DITTGSTVDMTESVGLTNMKATPLDAILTPRLSPTLY.

In one embodiment, the present application relates to a non-naturally occurring microorganism to produce baicalein comprising the sequence SEQ ID_NO 1 or a protein sequence having at least 70% identity thereof, respectively.

In one embodiment, the present application relates to a non-naturally occurring microorganism to produce baicalein comprising the sequence SEQ ID_NO 1 or a protein sequence having at least 80% identity thereof, respectively.

In one embodiment, the present application relates to a non-naturally occurring microorganism to produce baicalein comprising the sequence SEQ ID_NO 1 or a protein sequence having at least 90% identity thereof, respectively.

In a preferred embodiment, the present application relates to a non-naturally occurring microorganism to produce baicalein comprising the sequence SEQ ID_NO 1 or a protein sequence having at least 85% identity thereof, respectively.

In another embodiment, cytochrome P450 CYP82D1.2 (protein ID: ASW21051.1) from *Scutellaria baicalensis* is used with an n-terminal truncation of 25 amino acids. The resulting sequence CYPD1.2_TR (identified as SEQ ID_NO 2) comprises the amino acid primary sequence:

PKKSSLNAPPEAGGARFITGHLHLMDGRSASDKLPHINLGLLADQHGPIFTIRLGVHR
AVVVSSWELAKEIFTTHDTAVMARPRLIADDYLSYDGASLGFSPYGPYWREIRKLVT
TELYKLWEEKKDGSGRVLVDMKQWLGNLSLNLVSRMVVGKRFYGGDDSETTKRW
RGVMREFFQLIGQFIPGDGLPFLRWLDLGGFEKRTRDTAYELDKIIAMWLAEYRKRE
YSGDDKEQCFMALMLSLVQANPTLQLHYDADTIIKATCQVLISAASDTTTVILIWVISLL
LNNADVLKKVQEELDEQVGRERRVEESDISNLPYLQAVVKETMRLYPPAPFAGVRA
FSEDCTVGGYHIQKGTFLIVNLWKLHRDPRVWSDDALEFKPQRFFDKKVEVKGQDF
ELMPFGGGRRMCPGSNLGMHMVHFVLANILQAFDITTGSTVDMTESVGLTNMKATP
LDAILTPRLSPTLY

In one embodiment, the present application relates to a non-naturally occurring microorganism to produce baicalein comprising the sequence SEQ ID_NO 2 or a protein sequence having at least 70% identity thereof, respectively.

In one embodiment, the present application relates to a non-naturally occurring microorganism to produce baicalein comprising the sequence SEQ ID_NO 2 or a protein sequence having at least 80% identity thereof, respectively.

In one embodiment, the present application relates to a non-naturally occurring microorganism to produce baicalein comprising the sequence SEQ ID_NO 2 or a protein sequence having at least 90% identity thereof, respectively.

In a preferred embodiment, the present application relates to a non-naturally occurring microorganism to produce baicalein comprising the sequence SEQ ID_NO 2 or a protein sequence having at least 85% identity thereof, respectively.

In another embodiment, cytochrome P450 CYP82D1.2 (protein ID: ASW21051.1) from *Scutellaria baicalensis* is used with an n-terminal truncation of 25 amino acids and an n-terminal solubility tag. The resulting sequence CYPD1.2_TR_SOL (identified as SEQ ID_NO 3) comprises the amino acid primary sequence:

MPKKSSLNAPPEAGGARFITGHLHLMDGRSASDKLPHINLGLLADQHGPIFTIRLGV
HRAVVVSSWELAKEIFTTHDTAVMARPRLIADDYLSYDGASLGFSPYGPYWREIRKL
VTTELLSARRIELQRATRVREITQFTGELYKLWEEKKDGSGRVLVDMKQWLGNLSL
NLVSRMVVGKRFYGGDDSETTKRWRGVMREFFQLIGQFIPGDGLPFLRWLDLGGF
EKRTRDTAYELDKIIAMWLAEYRKREYSGDDKEQCFMALMLSLVQANPTLQLHYDA
DTIIKATCQVLISAASDTTTVILIWVISLLLNNADVLKKVQEELDEQVGRERRVEESDIS
NLPYLQAVVKETMRLYPPAPFAGVRAFSEDCTVGGYHIQKGTFLIVNLWKLHRDPR
VWSDDALEFKPQRFFDKKVEVKGQDFELMPFGGGRRMCPGSNLGMHMVHFVLA
NILQAFDITTGSTVDMTESVGLTNMKATPLDAILTPRLSPTLY

In one embodiment, the present application relates to a non-naturally occurring microorganism to produce baicalein comprising the sequence SEQ ID_NO 3 or a protein sequence having at least 70% identity thereof, respectively.

In one embodiment, the present application relates to a non-naturally occurring microorganism to produce baicalein comprising the sequence SEQ ID_NO 3 or a protein sequence having at least 80% identity thereof, respectively.

In one embodiment, the present application relates to a non-naturally occurring microorganism to produce baicalein comprising the sequence SEQ ID_NO 3 or a protein sequence having at least 90% identity thereof, respectively.

In a preferred embodiment, the present application relates to a non-naturally occurring microorganism to produce baicalein comprising the sequence SEQ ID_NO 3 or a protein sequence having at least 85% identity thereof, respectively.

B) Additionally, the enzymatic pathway in the non-naturally microorganism to produce baicalein from chrysin comprises a P450 reductase sequence from *Arabidopsis thaliana* and is used to ensure the correct functionality of cytochrome P450 CYP82D.1.2. The sequence of the P450 reductase is selected from the following sequences:
In one embodiment, cytochrome P450 reductase AtATR2 (Uniprot Number: Q9SUM3) (identified as AtATR2) from *Arabidopsis thaliana* is used. The following sequence AtATR2 (identified as SEQ ID_NO 4) comprises the amino acid primary sequence:

MSSSSSSSTSMIDLMAAIIKGEPVIVSDPANASAYESVAAELSSMLIENRQFAMIVTT
SIAVLIGCIVMLVWRRSGSGNSKRVEPLKPLVIKPREEEIDDGRKKVTIFFGTQTGTA
EGFAKALGEEAKARYEKTRFKIVDLDDYAADDDEYEEKLKKEDVAFFFLATYGDGE
PTDNAARFYKWFTEGNDRGEWLKNLKYGVFGLGNRQYEHFNKVAKVVDDILVEQG
AQRLVQVGLGDDDQCIEDDFTAWREALWPELDTILREEGDTAVATPYTAAVLEYRV
SIHDSEDAKFNDINMANGNGYTVFDAQHPYKANVAVKRELHTPESDRSCIHLEFDIA
GSGLTYETGDHVGVLCDNLSETVDEALRLLDMSPDTYFSLHAEKEDGTPISSSLPP
PFPPCNLRTALTRYACLLSSPKKSALVALAAHASDPTEAERLKHLASPAGKDEYSK
WVVESQRSLLEVMAEFPSAKPPLGVFFAGVAPRLQPRFYSISSSPKIAETRIHVTCA
LVYEKMPTGRIHKGVCSTWMKNAVPYEKSENCSSAPIFVRQSNFKLPSDSKVPIIMI
GPGTGLAPFRGFLQERLALVESGVELGPSVLFFGCRNRRMDFIYEEELQRFVESGA
LAELSVAFSREGPTKEYVQHKMMDKASDIWNMISQGAYLYVCGDAKGMARDVHRS
LHTIAQEQGSMDSTKAEGFVKNLQTSGRYLRDVW

In one embodiment, the present application relates to a non-naturally occurring microorganism to produce baicalein comprising the sequence SEQ ID_NO 4 or a protein sequence having at least 70% identity thereof, respectively.

In one embodiment, the present application relates to a non-naturally occurring microorganism to produce baicalein comprising the sequence SEQ ID_NO 4 or a protein sequence having at least 80% identity thereof, respectively.

In one embodiment, the present application relates to a non-naturally occurring microorganism to produce baicalein comprising the sequence SEQ ID_NO 4 or a protein sequence having at least 90% identity thereof, respectively.

In a preferred embodiment, the present application relates to a non-naturally occurring microorganism to produce baicalein comprising the sequence SEQ ID_NO 4 or a protein sequence having at least 85% identity thereof, respectively.

In another embodiment, cytochrome P450 reductase AtATR2 (Uniprot Number: Q9SUM3) (identified as AtATR2) from *Arabidopsis thaliana* is used with an n-terminal truncation of 72 amino acids. The resulting sequence, AtATR2_TR (identified as SEQ ID_NO 5) comprises the amino acid primary sequence:

RRSGSGNSKRVEPLKPLVIKPREEEIDDGRKKVTIFFGTQTGTAEGFAKALGEEAKA
RYEKTRFKIVDLDDYAADDDEYEEKLKKEDVAFFFLATYGDGEPTDNAARFYKWFT
EGNDRGEWLKNLKYGVFGLGNRQYEHFNKVAKVVDDILVEQGAQRLVQVGLGDD
DQCIEDDFTAWREALWPELDTILREEGDTAVATPYTAAVLEYRVSIHDSEDAKFNDI
NMANGNGYTVFDAQHPYKANVAVKRELHTPESDRSCIHLEFDIAGSGLTYETGDHV
GVLCDNLSETVDEALRLLDMSPDTYFSLHAEKEDGTPISSSLPPPFPPCNLRTALTR
YACLLSSPKKSALVALAAHASDPTEAERLKHLASPAGKDEYSKWVVESQRSLLEVM
AEFPSAKPPLGVFFAGVAPRLQPRFYSISSSPKIAETRIHVTCALVYEKMPTGRIHKG
VCSTWMKNAVPYEKSENCSSAPIFVRQSNFKLPSDSKVPIIMIGPGTGLAPFRGFLQ
ERLALVESGVELGPSVLFFGCRNRRMDFIYEEELQRFVESGALAELSVAFSREGPT
KEYVQHKMMDKASDIWNMISQGAYLYVCGDAKGMARDVHRSLHTIAQEQGSMDS
TKAEGFVKNLQTSGRYLRDVW

In one embodiment, the present application relates to a non-naturally occurring microorganism to produce baicalein comprising the sequence SEQ ID_NO 5 or a protein sequence having at least 70% identity thereof, respectively.

In one embodiment, the present application relates to a non-naturally occurring microorganism to produce baicalein comprising the sequence SEQ ID_NO 5 or a protein sequence having at least 80% identity thereof, respectively.

In one embodiment, the present application relates to a non-naturally occurring microorganism to produce baicalein comprising the sequence SEQ ID_NO 5 or a protein sequence having at least 90% identity thereof, respectively.

In a preferred embodiment, the present application relates to a non-naturally occurring microorganism to produce baicalein comprising the sequence SEQ ID_NO 5 or a protein sequence having at least 85% identity thereof, respectively.

In another embodiment, cytochrome P450 reductase CPR1 (Uniprot Number: Q9SB48.1) (identified as CPR1) from *Arabidopsis thaliana* is used. The following sequence CPR1 (identified as SEQ ID_NO 6) comprises the amino acid primary sequence:

In one embodiment, the present application relates to a non-naturally occurring microorganism to produce baicalein comprising the sequence SEQ ID_NO 6 or a protein sequence having at least 70% identity thereof, respectively.

In one embodiment, the present application relates to a non-naturally occurring microorganism to produce baicalein comprising the sequence SEQ ID_NO 6 or a protein sequence having at least 80% identity thereof, respectively.

In one embodiment, the present application relates to a non-naturally occurring microorganism to produce baicalein comprising the sequence SEQ ID_NO 6 or a protein sequence having at least 90% identity thereof, respectively.

In a preferred embodiment, the present application relates to a non-naturally occurring microorganism to produce baicalein comprising the sequence SEQ ID_NO 6 or a protein sequence having at least 85% identity thereof, respectively.

In another embodiment, cytochrome P450 reductase CPR1 (Uniprot Number: Q9SB48.1) (identified as CPR1) from *Arabidopsis thaliana* is used with an n-terminal truncation of 42 amino acids. The resulting sequence, CPR1_TR (identified as SEQ ID_NO 7) comprises the amino acid primary sequence:

VVLVIATTSLALVAGFVVLLWKKTTADRSGELKPLMIPKSLMAKDEDDDLDLGSGKT
RVSIFFGTQTGTAEGFAKALSEEIKARYEKAAVKVIDLDDYAADDDQYEEKLKKETLA
FFCVATYGDGEPTDNAARFYKWFTEENERDIKLQQLAYGVFALGNRQYEHFNKIGI
VLDEELCKKGAKRLIEVGLGDDDQSIEDDFNAWKESLWSELDKLLKDEDDKSVATP
YTAVIPEYRVVTHDPRFTTQKSMESNVANGNTTIDIHHPCRVDVAVQKELHTHESD
RSCIHLEFDISRTGITYETGDHVGVYAENHVEIVEEAGKLLGHSLDLVFSIHADKEDG
SPLESAVPPPFPGPCTLGTGLARYADLLNPPRKSALVALAAYATEPSEAEKLKHLTS
PDGKDEYSQWIVASQRSLLEVMAAFPSAKPPLGVFFAAIAPRLQPRYYSISSSPRLA
PSRVHVTSALVYGPTPTGRIHKGVCSTWMKNAVPAEKSHECSGAPIFIRASNFKLP
SNPSTPIVMVGPGTGLAPFRGFLQERMALKEDGEELGSSLLFFGCRNRQMDFIYED
ELNNFVDQGVISELIMAFSREGAQKEYVQHKMMEKAAQVWDLIKEEGYLYVCGDA
KGMARDVHRTLHTIVQEQEGVSSSEAEAIVKKLQTEGRYLRDVW

In one embodiment, the present application relates to a non-naturally occurring microorganism to produce baicalein comprising the sequence SEQ ID_NO 7 or a protein sequence having at least 70% identity thereof, respectively.

In one embodiment, the present application relates to a non-naturally occurring microorganism to produce baicalein comprising the sequence SEQ ID_NO 7 or a protein sequence having at least 80% identity thereof, respectively.

In one embodiment, the present application relates to a non-naturally occurring microorganism to produce baicalein comprising the sequence SEQ ID_NO 7 or a protein sequence having at least 90% identity thereof, respectively.

In a preferred embodiment, the present application relates to a non-naturally occurring microorganism to produce baicalein comprising the sequence SEQ ID_NO 7 or a protein sequence having at least 85% identity thereof, respectively.

### Transformation of the Microorganisms

In a preferred embodiment, the microorganism is *Escherichia coli* (selected from *Escherichia coli BLR (DE3), Escherichia coli Rosetta2 (DE3), Escherichia coli T7 Express or Escherichia coli BL21 (DE3))* and it is transformed using genetic engineering tools to insert the desired sequences.

In a preferred embodiment, *Escherichia coli* is modified using SEQ ID_3 and SEQ ID_NO 4 to produce baicalein.

In another embodiment, *Escherichia coli* is modified using SEQ ID_3 and SEQ ID_NO 6 to produce baicalein.

### Genetic Engineering Using Plasmid Shuttle Vectors

In an embodiment, the genes which enable the expression of enzymes used to produce baicalein are transformed into *Escherichia coli* via a shuttle vector plasmid.

The shuttle vector plasmid comprises one or more specific *Escherichia coli* promoters, a T7 IPTG inducible promoter, one or more multiple cloning sites for the insertion of single genes or gene clusters, one or more terminators, one or more resistance cassettes (selected from ampicillin resistance (AmpR), kanamycin resistance (KanaR), chloramphenicol resistance (CamR), spectinomycin resistance (SpecR), tetracycline resistance (TetR)) and an *Escherichia coli* Origin of replication (OR).

In an embodiment, the shuttle vectors comprising the genes are transformed via electroporation and subsequently plated on agar plates containing one or more of the following antibiotics ampicillin, kanamycin, chloramphenicol, tetracycline and spectinomycin. The detection of the successful transformation of gene cassettes is accomplished via PCR and sequencing. The gene transcription and enzyme translation and activity are determined by transcriptome, proteome and metabolome analysis.

In a preferred embodiment, the shuttle vectors comprise SEQ ID_3 and SEQ ID_NO 4 to produce baicalein.

In another embodiment, the shuttle vectors comprise SEQ ID_3 and SEQ ID_NO 6 to produce baicalein.

### Genome Modification Using CRISPR-Cas System

In an embodiment, *Escherichia coli* is genetically engineered via the CRISPR-Cas system. This technique enables deletions, insertions or point mutations in the genomic DNA of the microorganism. The system can recognize a protospacer adjacent motif (PAM) next to the genomic target site and it generates a double-strand break. This is followed by DNA repair initiation through homologous directed repair if an exogenous homologous donor sequence is present (Zhao et. al. 2016). If the exogenous homologous donor sequence flanks the desired gene sequence, this sequence is stably introduced into the *Escherichia coli* genome.

In an embodiment, *Escherichia coli* can be modified genetically via the CRISPR-Cas system. Plasmids containing a Cas protein and a corresponding guide RNA are transformed together with the gene sequence flanked with the DNA fragments complementary to the insertion site. The plasmids are transformed by electroporation and subsequently spread on agar plates, supplemented with antibiotics. By colony PCR, the target sequence is amplified with the help of the corresponding primers and individual positive colonies are then identified with agarose gel electrophoresis and sequencing.

### Genome Modification via Lambda Red

In an embodiment, *Escherichia coli* is genetically modified via the phage Lambda-derived Red recombination system. This technique enables deletions, insertions or point mutations in the genomic DNA of *Escherichia coli.*

In an embodiment, a plasmid is used comprising the bacteriophage lambda red components. The repair template essential for the homologous recombination is designed to have homologous arms to the genomic DNA of the microorganism where the mutation is incorporated as well as containing the desired mutation. If the homologous arms flank the desired gene sequence, this sequence is stably introduced into the *Escherichia coli* genome. Both the plasmid and the repair template are transformed into *Escherichia coli.*

In an embodiment, the Lambda-derived Red recombination plasmids are transformed by electroporation into *Escherichia coli* and subsequently spread on agar plates, supplemented with antibiotics. By colony PCR, the target sequence is amplified with assistance of the corresponding primers and individual positive colonies are then identified with agarose gel electrophoresis and sequencing.

### Genome Modification via Homologous Recombination (HR).

Homologous recombination is used as a method for the introduction of genetic material to make the non-natural occurring microorganism. The genetic material for the homologous recombination contains a high degree of homology to the genetic sequence of the microorganism. Through an endogenous in cell mechanism, the region with a high degree of homology gets built into the genome creating a non-naturally occurring microorganism. The homologous regions are designed in a way that they flank a specific sequence region. This sequence region gets incorporated into the genome of the bacteria through homologous recombination.

In an embodiment, homologous gene sequence flanking the gene by electroporation into the microorganism and subsequently spread on agar plates, which have been supplemented with antibiotics. By colony PCR, the target sequence is amplified with the help of the corresponding primers and individual positive colonies are then identified with agarose gel electrophoresis and sequencing.

In a preferred embodiment the microorganism is *Zymomonas mobilis ZM4* and it is transformed using genetic engineering tools to insert the desired sequences.

In a preferred embodiment, *Zymomonas mobilis ZM4* is modified by inserting SEQ ID_3 and SEQ ID_NO 4 to produce baicalein.

In another embodiment, *Zymomonas mobilis ZM4* is modified by inserting SEQ ID_3 and SEQ ID_NO 6 to produce baicalein.

### Genetic Engineering Using Plasmid Shuttle Vectors

In an embodiment the genes, which enable the expression of enzymes used for the production of baicalein are transformed into *Zymomonas mobilis* via a shuttle vector plasmid.

The shuttle vector plasmid can contain one or more specific promoters, one or more multiple cloning sites for the insertion of single genes or gene clusters, one or more terminators, one or more resistance cassettes (selected from ampicillin resistance (AmpR), kanamycin resistance (KanaR), chloramphenicol resistance (CamR), spectinomycin resistance (SpecR), tetracycline resistance (TetR)) and an *Zymomonas mobilis* Origin of Replication (OR).

In an embodiment, the shuttle vectors with the incorporated genes are transformed into *Zymomonas mobilis* via electroporation and subsequently plated on agar plates containing one or more of the following antibiotics ampicillin, kanamycin, chloramphenicol, tetracycline and spectinomycin. Detection of the successful transformation of gene cassettes is accomplished via PCR and sequencing. The gene transcription and enzyme translation and activity are determined by transcriptome, proteome and metabolome analysis.

In a preferred embodiment, the shuttle vectors comprise SEQ ID_3 and SEQ ID_NO 4 to produce baicalein.

In another embodiment, the shuttle vectors comprise SEQ ID_3 and SEQ ID_NO 6 to produce baicalein.

### Genome engineering using CRISPR-Cas system

In an embodiment, the microorganism can be genetically engineered via the CRISPR-Cas system. This technique enables deletions, insertions or point mutations in the genomic DNA of the microorganism.

In an embodiment the gram-negative bacterial strain *Zymomonas mobilis* is a genetically engineered microorganism via the use of the endogenous CRISPR-Cas system (type III) in order to introduce deletions, insertions or point mutations in the genomic DNA of the microorganism (Zheng et. al. 2019).

### Genome Modification via Lambda Red

*Zymomonas mobilis* is genetically modified via the phage Lambda-derived Red recombination system. This technique enables deletions, insertions or point mutations in the genomic DNA of *Zymomonas mobilis.*

In an embodiment, a plasmid is used comprising the bacteriophage lambda red components. The repair template essential for the homologous recombination is designed to have homologous arms to the genomic DNA of the microorganism where the mutation is incorporated as well as containing the desired mutation. If the homologous arms flank the desired gene sequence, this sequence is stably introduced into the *Zymomonas mobilis* genome. Both the plasmid and the repair template are transformed into *Zymomonas mobilis.*

In an embodiment, the Lambda-derived Red recombination plasmids are transformed by electroporation into the microorganism and subsequently spread on agar plates, which have been supplemented with antibiotics. By colony PCR, the target sequence is amplified with the help of the corresponding primers and individual positive colonies are then identified with agarose gel electrophoresis and sequencing.

In a preferred embodiment the microorganism is *Saccharomyces Cerevisiae* and it is transformed using genetic engineering tools to insert the desired sequences.

In a preferred embodiment, *Saccharomyces Cerevisiae* is modified by inserting SEQ ID_3 and SEQ ID_NO 4 to produce baicalein.

In another embodiment, *Saccharomyces Cerevisiae* is modified by inserting SEQ ID_3 and SEQ ID_NO 6 to produce baicalein.

### Genome Modification via Homologous Directed Repair (HDR).

Homologous directed repair is used as a method for the introduction of genetic material to make the non-natural occurring microorganism. The genetic material for the homologous directed repair contains a high degree of homology to the genetic sequence of the microorganism. Through an endogenous in cell mechanism the region with a high degree of homology gets built into the genome creating a non-naturally occurring microorganism. The homologous regions are designed in a way that they flank a specific sequence region. This sequence region gets incorporated into the genome of the bacteria through homologous recombination.

In an embodiment, the incorporated region contains FRT-sequences from *Saccharomyces cerevisiae.* The FRT sequence is used as a FLP-FRT site-specific recombination system. Gene fragments flanking two FRT sequences are incorporated by the *Saccharomyces cerevisiae* FLT gene coding for the FLP recombinase enzyme (Zou et al 2012).

### Genetic Engineering Using Plasmid Shuttle Vectors

In an embodiment, the genes which enable the expression of enzymes used to produce baicalein are transformed into *Saccharomyces Cerevisiae* via a shuttle vector plasmid.

The shuttle vector plasmid comprises one or more specific *Saccharomyces Cerevisiae* promoters, one or more multiple cloning sites for the insertion of single genes or gene clusters, one or more terminators, one or more resistance cassettes (selected from ampicillin resistance (AmpR), kanamycin resistance (KanaR), chloramphenicol resistance (CamR), spectinomycin resistance (SpecR), tetracycline resistance (TetR)) and an *Saccharomyces Cerevisiae* Origin of Replication (OR).

In an embodiment, the shuttle vectors comprising the genes (cytochrome P450 CYP82D1.2 and cytochrome P450 reductase) are transformed via electroporation and subsequently plated on agar plates containing one or more of the following antibiotics ampicillin, kanamycin, chloramphenicol, tetracycline and spectinomycin. The detection of the successful transformation of gene cassettes is accomplished via PCR and sequencing. The gene transcription and enzyme translation and activity are determined by transcriptome, proteome and metabolome analysis.

In a preferred embodiment, the shuttle vectors comprise SEQ ID_3 and SEQ ID_NO 4 to produce baicalein.

In another embodiment, the shuttle vectors comprise SEQ ID_3 and SEQ ID_NO 6 to produce baicalein.

### Genome Engineering Using CRISPR-Cas System

In an embodiment, *Saccharomyces Cerevisiae* can be genetically modified via the CRISPR-Cas system. This technique enables deletions, insertions or point mutations in the genomic DNA of the microorganism. The system is able to recognize a protospacer adjacent motif (PAM) next to the genomic target site and generate a double-strand break. This is followed by DNA repair initiation through homologous directed repair if an exogenous homologous donor sequence is present. If the exogenous homologous donor sequence flanks the desired gene sequence, this sequence is stably introduced into the *Saccharomyces Cerevisiae* genome.

### Genome Modification via Homologous Directed Repair (HDR).

Homologous directed repair is used as a method for the introduction of genetic material to make the non-natural occurring bacterial. The genetic material for the homologous directed repair contains a high degree of homology to the genetic sequence of the microorganism. Through an endogenous in cell mechanism the region with a high degree of homology gets built into the genome creating a non-naturally occurring microorganism. The homologous regions are designed in a way that they flank a specific sequence region. This sequence region gets incorporated into the genome of the bacteria through homologous recombination.

In a preferred embodiment the microorganism is *Clostridium autoethanogenum* and it is transformed using genetic engineering tools to insert the desired sequences.

In a preferred embodiment, *Clostridium autoethanogenum* is modified by inserting SEQ ID_3 and SEQ ID_NO 4 to produce baicalein.

In another embodiment, *Clostridium autoethanogenum* is modified by inserting SEQ ID_3 and SEQ ID_NO 6 to produce baicalein.

### Genetic Engineering Using Conjugation

In an embodiment, plasmids containing the genes are transformed into an *Escherichia coli* donor strain and then transferred to *Clostridium autoethanogenum* by conjugation. Thiamphenicol (7.5 µg/ml) is used to counter select against the *Escherichia coli* donor strain (Liew et. al. 2017).

In a preferred embodiment, the plasmids comprise the genes SEQ ID_3 and SEQ ID_NO 4 to produce baicalein.

In another embodiment, the plasmids comprise the genes SEQ ID_3 and SEQ ID_NO 6 to produce baicalein.

### Genome Engineering Using CRISPR-Cas System

In an embodiment, *Clostridium autoethanogenum* can be genetically engineered via the CRISPR-Cas system (Nagaraju et. al. 2016). This technique enables deletions, insertions or point mutations in the genomic DNA of the microorganism. The system is able to recognize a protospacer adjacent motif (PAM) next to the genomic target site and generate a double-strand break. This is followed by DNA repair initiation through homologous directed repair if an exogenous homologous donor sequence is present.

In an embodiment, *Closteridium autoethanogenum* can be modified genetically via the CRISPR-Cas system. Plasmids containing a Cas protein and a corresponding guide RNA are transformed together with the gene sequence flanked with the DNA fragments complementary to the insertion site. The plasmids are transformed by electroporation and subsequently spread on agar plates, which have been supplemented with antibiotics. By colony PCR, the target sequence is amplified with the help of the corresponding primers and individual positive colonies are then identified with agarose gel electrophoresis and sequencing.

### Verification of Transformation

In an embodiment, the shuttle vectors with the incorporated genes are transformed into the microorganism via electroporation and subsequently plated on agar plates containing one or more of the following antibiotics ampicillin, kanamycin, chloramphenicol, tetracycline and spectinomycin. Detection of the successful transformation of gene cassettes is accomplished via PCR and sequencing. The gene transcription and enzyme translation and activity are determined by transcriptome, proteome and metabolome analysis.

### Media Supplementation

The production of baicalein is ensured by the addition of the chrysin precursor molecule into the culture medium.

In an embodiment, chrysin is fed to the culture medium at a final concentration between 0.1 g/L and 6 g/L. Chrysin can be added at the time of inoculation between 1 hour and 18 hours after inoculation.

The production of baicalein is ensured by the addition of the chrysin, the precursor molecule, into the culture medium.

In an embodiment, chrysin is fed to the culture medium at a final concentration between 0.1 g/L and 6 g/L. Chrysin can be added at the time of inoculation between 1 hour and 18 hours after inoculation.

In a preferred embodiment, Chrysin fed to the culture medium at a final concentration of 0.4 g/L. Chrysin can be added at the time of 4 hours after inoculation.

### Confirmation of Baicalein in Solution

The genetically engineered microorganism (for example any before mentioned *Escherichia coli, Zymomonas mobilis, Saccharomyces cerevisiae or Clostridium autoethanogenum)* is cultivated and grown in cell medium.

In an embodiment, the cells are disrupted and the precursor molecule chrysin is added to the disrupted cell medium. The disrupted cells are incubated together with chrysin for 4 hours at 37°C for the enzymatic transformation to baicalein.

In an embodiment, the cells are disrupted and the precursor molecules of chrysin are added to the disrupted cell medium. The disrupted cells are incubated together with chrysin for 4 hours at 37°C for the enzymatic transformation to baicalein.

In another embodiment, a whole cell approach is used where undisrupted cells are cultivated in culture medium and the precursor molecules of chrysin are added to the culture medium. The precursor molecules are absorbed by the genetically engineered microorganisms and enzymatically transformed into baicalein, which again is released from the genetically engineered microorganisms into the culture medium. The cells therefore do not have to be disrupted and the product baicalein is detected in culture medium.

In another embodiment, a whole cell approach is used where undisrupted cells are cultivated in culture medium and the precursor molecules of chrysin are added to the culture medium. The precursor molecules are absorbed by the genetically modified microorganisms and enzymatically transformed into baicalein, which again is released from the genetically modified microorganisms into the culture medium. The cells therefore do not have to be disrupted and the product, baicalein, is detected in culture medium.

Identification and quantification of baicalein can be performed by several methods combining chromatographic separation; for example LC, HPLC, UHPLC or GC, and mass sensitive or photo optical detection. Chromatographic methods can include liquid (MeOH, ACN, hexan, water, acetic acid and others) or gaseous mobile phases (H2, He, N2, Ar) and liquid or solid stationary phases including silica gel, polydimethylsiloxane or reversed phase materials. Detection of baicalein and related compounds can be achieved by using MS or MS/MS including sector mass spectrometry, time-of-flight mass spectrometry, the use of quadrupole mass analyzer, three-dimensional quadrupole ion trap, cylindrical ion trap, linear quadrupole ion trap, orbitrap or fourier transform ion cyclotron resonance and the use of an adequate detector, including electron multiplier systems, faraday cups, ion-to-photon detectors, microchannel plate detectors or inductive detectors. Other detection methods like UV-absorption, fluorescence, charged aerosol detector, evaporative light scattering detector, flame ionization detector, flame photometric detector, nitrogen phosphorus detector, atomic-emission detector, refractive index detector, radio flow detector, conductivity monitor, thermal conductivity detector, electron capture detector and photoionization detectors or combination of those principles can be applied. Baicalein can also be detected by using chemical reactions, the use of appropriate stains like iodine vapor, iodoplatinate, marquis reagent, nihydrin, HNO₃-atmosphere, NNCD-reagent, PDAB-TS, TACOT, TCBI, vanillin reagents, Van Urk reagent or xanthydrol and the use of an authentic reference substance (Barker et al. 2012; Mulga et al. 2012).

Quantification can be achieved by the use of an internal standard containing baicalein. By the addition of a known amount of these substances prior to sample preparation, it is possible to calculate the actual amount of baicalein and related compounds in a given sample (Barker et al. 2013).

### Confirmation of Baicalein in Solution

The genetically modified microorganism (For example: *Escherichia coli, Zymomonas mobilis, Saccharomyces cerevisiae, Clostridium autoethanogenum)* is cultivated and grown in cell medium.

In an embodiment the cells are disrupted and the precursor molecules, chrysin, are added to the disrupted cell medium. The disrupted cells are incubated together with chrysin for 4 hours at 37°C for the enzymatic transformation to baicalein.

In another embodiment a whole cell approach is used where undisrupted cells are cultivated in culture medium and the precursor molecules, chrysin, are added to the culture medium. The precursor molecules are absorbed by the genetically engineered microorganisms and enzymatically transformed into baicalein, which again is released from the genetically modified microorganisms into the culture medium. Therefore, the cells do not have to be disrupted and the product baicalein is detected in culture medium.

Identification and quantification of baicalein can be performed by several methods combining chromatographic separation like LC, HPLC, UHPLC or GC and mass sensitive or photo optical detection. Chromatographic methods can include liquid (MeOH, ACN, hexan, water, acetic acid and others) or gaseous mobile phases (H2, He, N2, Ar) and liquid or solid stationary phases including silica gel, polydimethylsiloxane or reversed phase materials.

Detection of baicalein and related compounds can be achieved by using MS or MS/MS including sector mass spectrometry, time-of-flight mass spectrometry, the use of quadrupole mass analyzer, three-dimensional quadrupole ion trap, cylindrical ion trap, linear quadrupole ion trap, orbitrap or fourier transform ion cyclotron resonance and the use of an adequate detector, including electron multiplier systems, faraday cups, ion-to-photon detectors, microchannel plate detectors or inductive detectors. Other detection methods like UV-absorption, fluorescence, charged aerosol detector, evaporative light scattering detector, flame ionization detector, flame photometric detector, nitrogen phosphorus detector, atomic-emission detector, refractive index detector, radio flow detector, conductivity monitor, thermal conductivity detector, electron capture detector and photoionization detectors or combination of those principles can be applied.

Baicalein can also be detected by using chemical reactions, the use of appropriate stains like iodine vapor, iodoplatinate, marquis reagent, nihydrin, HNO₃-atmosphere, NNCD-reagent, PDAB-TS, TACOT, TCBI, vanillin reagents, Van Urk reagent or xanthydrol and the use of an authentic reference substance (Barker et al. 2012; Mulga et al. 2012).

Quantification can be achieved using an internal standard containing baicalein. By the addition of a known amount of these substances prior to sample preparation it is possible to calculate the actual amount of baicalein and related compounds in a given sample (Barker et al. 2013).

In an embodiment, the baicalein produced by the non-naturally occurring microorganism is used to obtain baicalein through glycosynthases that have been developed and can form glycosidic bonds.

With the above context, the following consecutively numbered embodiments provide further specific aspects of the invention:
1. A non-natural occurring microorganism which is able to biosynthetically produce baicalein (5,6,7-trihydroxyflavone), wherein said microorganism has been modified to comprise an enzymatic pathway for the biosynthetic production of baicalein.
2. A non-natural occurring microorganism which is able to biosynthetically produce baicalein according to embodiment 1, wherein the precursor for the biosynthetic production of baicalein is chrysin.
3. A non-natural occurring microorganism which is able to biosynthetically produce baicalein according to embodiments 1 and 2, wherein said enzymatic pathway comprises at least one cytochrome and at least one cytochrome reductase.
4. A non-natural occurring microorganism which is able to biosynthetically produce baicalein according to embodiments 1 to 3, wherein said microorganism has been modified to comprise cytochrome P450 and cytochrome P450 reductase.
5. A non-natural occurring microorganism which is able to biosynthetically produce baicalein according to embodiments 1 to 4, wherein said microorganism has been modified to comprise cytochrome P450 CYP82D1.2 and cytochrome P450 reductase.
6. A non-natural occurring microorganism which is able to biosynthetically produce baicalein according to embodiments 1 to 5, wherein cytochrome P450 CYP82D1.2 is overexpressed in said microorganism for the biosynthetic production of baicalein.
7. A non-natural occurring microorganism which is able to biosynthetically produce baicalein according to embodiments 1 to 6, wherein the cytochrome P450 CYP82D1.2 is selected from SEQ ID_NO 1 or a protein sequence having at least 70% identity thereof, SEQ ID_NO 2 or a protein sequence having at least 70% identity thereof, and SEQ ID_NO 3 or a protein sequence having at least 70% identity thereof, and the cytochrome P450 reductase is selected from SEQ ID_NO 4 or a protein sequence having at least 70% identity thereof, SEQ ID_NO 5 or a protein sequence having at least 70% identity thereof, SEQ ID_NO 6 or a protein sequence having at least 70% identity thereof, and SEQ ID_NO 7 or a protein sequence having at least 70% identity thereof.
8. A non-natural occurring microorganism according to embodiments 1 to 7, wherein SEQ ID_NO 1, SEQ ID_NO 2, SEQ ID_NO 3, SEQ ID_NO 4, SEQ ID_NO 5, SEQ ID_NO 6 and SEQ ID_NO 7 have at least an 85% of identity to any of the sequences SEQ ID_NO 1-7.
9. A non-natural occurring microorganism according to embodiments 1 to 8, wherein said microorganism is genetically modified with SEQ ID_NO 3 or a protein sequence having at least 70% identity thereof, and SEQ ID_NO 4 or a protein sequence having at least 70% identity thereof.
10. A non-natural occurring microorganism according to embodiments 1 to 9, wherein said microorganism is genetically modified with SEQ ID_NO 3 or a protein sequence having at least 70% identity thereof, and SEQ ID_NO 6 or a protein sequence having at least 70% identity thereof.
11. A non-natural occurring microorganism according to embodiments 1 to 10, wherein said microorganism is a bacterium.
12. A non-natural occurring microorganism according to embodiment 11, wherein said bacterium is from the *Escherichia* genus.
13. A non-natural occurring microorganism according to embodiment 12 wherein said bacteria is selected between *Escherichia coli BLR (DE3), Escherichia coli Rosetta2 (DE3), Escherichia coli T7* and *Express Escherichia coli BL21 (DE3).*
14. A non-natural occurring microorganism according to embodiment 11 wherein said bacterium is from the *Zymomonas* genus.
15. A non-natural occurring microorganism according to embodiment 14 wherein said bacteria is *Zymomonas mobilis.*
16. A non-natural occurring microorganism according to embodiment 11, wherein said bacterium is from the *Clostridium* genus.
17. A non-natural occurring microorganism according to embodiment 16, wherein said bacterium is from the *Clostridium autoethanogenum.*
18. A non-natural occurring microorganism according to embodiments 1 to 10, wherein said microorganism is a eukaryote.
19. A non-natural occurring microorganism according to embodiment 18, wherein said eukaryote is a *Saccharomyces.*
20. A non-natural occurring microorganism according to embodiment 19 , wherein said eukaryote is *Saccharomyces Cerevisiae*
21. A method to produce baicalein, comprising a non-natural occurring microorganism according to embodiments 1-20, and in particular, which has been modified to comprise cytochrome P450 CYP82D1.2 and cytochrome P450 reductase.
22. Use of a non-natural occurring microorganism according to embodiments 1-20 for biosynthetic production of baicalein from the precursor molecule chrysin, and in particular wherein said microorganism has been modified to comprise cytochrome P450 CYP82D1.2 and cytochrome P450 reductase.

SEQUENCES

SEQ ID_NO 1:
MELSSVIYGAIALLSLFYCYLHFSKPKKSSLNAPPEAGGARFITGHLHLMDGRSASDK
LPHINLGLLADQHGPIFTIRLGVHRAVVVSSWELAKEIFTTHDTAVMARPRLIADDYLS
YDGASLGFSPYGPYWREIRKLVTTELYKLWEEKKDGSGRVLVDMKQWLGNLSLNLV
SRMVVGKRFYGGDDSETTKRWRGVMREFFQLIGQFIPGDGLPFLRWLDLGGFEKR
TRDTAYELDKIIAMWLAEYRKREYSGDDKEQCFMALMLSLVQANPTLQLHYDADTIIK
ATCQVLISAASDTTTVILIWVISLLLNNADVLKKVQEELDEQVGRERRVEESDISNLPY
LQAVVKETMRLYPPAPFAGVRAFSEDCTVGGYHIQKGTFLIVNLWKLHRDPRVWSD
DALEFKPQRFFDKKVEVKGQDFELMPFGGGRRMCPGSNLGMHMVHFVLANILQAF
DITTGSTVDMTESVGLTNMKATPLDAILTPRLSPTLY

SEQ ID_NO 2:
PKKSSLNAPPEAGGARFITGHLHLMDGRSASDKLPHINLGLLADQHGPIFTIRLGVHR
AVVVSSWELAKEIFTTHDTAVMARPRLIADDYLSYDGASLGFSPYGPYWREIRKLVT
TELYKLWEEKKDGSGRVLVDMKQWLGNLSLNLVSRMVVGKRFYGGDDSETTKRW
RGVMREFFQLIGQFIPGDGLPFLRWLDLGGFEKRTRDTAYELDKIIAMWLAEYRKRE
YSGDDKEQCFMALMLSLVQANPTLQLHYDADTIIKATCQVLISAASDTTTVILIWVISLL
LNNADVLKKVQEELDEQVGRERRVEESDISNLPYLQAVVKETMRLYPPAPFAGVRA
FSEDCTVGGYHIQKGTFLIVNLWKLHRDPRVWSDDALEFKPQRFFDKKVEVKGQDF
ELMPFGGGRRMCPGSNLGMHMVHFVLANILQAFDITTGSTVDMTESVGLTNMKATP
LDAILTPRLSPTLY

SEQ ID_NO 3:
MPKKSSLNAPPEAGGARFITGHLHLMDGRSASDKLPHINLGLLADQHGPIFTIRLGV
HRAVVVSSWELAKEIFTTHDTAVMARPRLIADDYLSYDGASLGFSPYGPYWREIRKL
VTTELLSARRIELQRATRVREITQFTGELYKLWEEKKDGSGRVLVDMKQWLGNLSL
NLVSRMVVGKRFYGGDDSETTKRWRGVMREFFQLIGQFIPGDGLPFLRWLDLGGF
EKRTRDTAYELDKIIAMWLAEYRKREYSGDDKEQCFMALMLSLVQANPTLQLHYDA
DTIIKATCQVLISAASDTTTVILIWVISLLLNNADVLKKVQEELDEQVGRERRVEESDIS

NLPYLQAVVKETMRLYPPAPFAGVRAFSEDCTVGGYHIQKGTFLIVNLWKLHRDPR
VWSDDALEFKPQRFFDKKVEVKGQDFELMPFGGGRRMCPGSNLGMHMVHFVLA
NILQAFDITTGSTVDMTESVGLTNMKATPLDAILTPRLSPTLY

SEQ ID_NO 4:
MSSSSSSSTSMIDLMAAIIKGEPVIVSDPANASAYESVAAELSSMLIENRQFAMIVTT
SIAVLIGCIVMLVWRRSGSGNSKRVEPLKPLVIKPREEEIDDGRKKVTIFFGTQTGTA
EGFAKALGEEAKARYEKTRFKIVDLDDYAADDDEYEEKLKKEDVAFFFLATYGDGE
PTDNAARFYKWFTEGNDRGEWLKNLKYGVFGLGNRQYEHFNKVAKVVDDILVEQG
AQRLVQVGLGDDDQCIEDDFTAWREALWPELDTILREEGDTAVATPYTAAVLEYRV
SIHDSEDAKFNDINMANGNGYTVFDAQHPYKANVAVKRELHTPESDRSCIHLEFDIA
GSGLTYETGDHVGVLCDNLSETVDEALRLLDMSPDTYFSLHAEKEDGTPISSSLPP
PFPPCNLRTALTRYACLLSSPKKSALVALAAHASDPTEAERLKHLASPAGKDEYSK
WVVESQRSLLEVMAEFPSAKPPLGVFFAGVAPRLQPRFYSISSSPKIAETRIHVTCA
LVYEKMPTGRIHKGVCSTWMKNAVPYEKSENCSSAPIFVRQSNFKLPSDSKVPIIMI
GPGTGLAPFRGFLQERLALVESGVELGPSVLFFGCRNRRMDFIYEEELQRFVESGA
LAELSVAFSREGPTKEYVQHKMMDKASDIWNMISQGAYLYVCGDAKGMARDVHRS
LHTIAQEQGSMDSTKAEGFVKNLQTSGRYLRDVW

SEQ ID_NO 5:
RRSGSGNSKRVEPLKPLVIKPREEEIDDGRKKVTIFFGTQTGTAEGFAKALGEEAKA
RYEKTRFKIVDLDDYAADDDEYEEKLKKEDVAFFFLATYGDGEPTDNAARFYKWFT
EGNDRGEWLKNLKYGVFGLGNRQYEHFNKVAKVVDDILVEQGAQRLVQVGLGDD
DQCIEDDFTAWREALWPELDTILREEGDTAVATPYTAAVLEYRVSIHDSEDAKFNDI
NMANGNGYTVFDAQHPYKANVAVKRELHTPESDRSCIHLEFDIAGSGLTYETGDHV
GVLCDNLSETVDEALRLLDMSPDTYFSLHAEKEDGTPISSSLPPPFPPCNLRTALTR
YACLLSSPKKSALVALAAHASDPTEAERLKHLASPAGKDEYSKWVVESQRSLLEVM
AEFPSAKPPLGVFFAGVAPRLQPRFYSISSSPKIAETRIHVTCALVYEKMPTGRIHKG
VCSTWMKNAVPYEKSENCSSAPIFVRQSNFKLPSDSKVPIIMIGPGTGLAPFRGFLQ
ERLALVESGVELGPSVLFFGCRNRRMDFIYEEELQRFVESGALAELSVAFSREGPT
KEYVQHKMMDKASDIWNMISQGAYLYVCGDAKGMARDVHRSLHTIAQEQGSMDS
TKAEGFVKNLQTSGRYLRDVW

SEQ ID_NO 6:














SEQ ID_NO 7:
VVLVIATTSLALVAGFVVLLWKKTTADRSGELKPLMIPKSLMAKDEDDDLDLGSGKT
RVSIFFGTQTGTAEGFAKALSEEIKARYEKAAVKVIDLDDYAADDDQYEEKLKKETLA
FFCVATYGDGEPTDNAARFYKWFTEENERDIKLQQLAYGVFALGNRQYEHFNKIGI
VLDEELCKKGAKRLIEVGLGDDDQSIEDDFNAWKESLWSELDKLLKDEDDKSVATP
YTAVIPEYRVVTHDPRFTTQKSMESNVANGNTTIDIHHPCRVDVAVQKELHTHESD
RSCIHLEFDISRTGITYETGDHVGVYAENHVEIVEEAGKLLGHSLDLVFSIHADKEDG
SPLESAVPPPFPGPCTLGTGLARYADLLNPPRKSALVALAAYATEPSEAEKLKHLTS
PDGKDEYSQWIVASQRSLLEVMAAFPSAKPPLGVFFAAIAPRLQPRYYSISSSPRLA
PSRVHVTSALVYGPTPTGRIHKGVCSTWMKNAVPAEKSHECSGAPIFIRASNFKLP
SNPSTPIVMVGPGTGLAPFRGFLQERMALKEDGEELGSSLLFFGCRNRQMDFIYED
ELNNFVDQGVISELIMAFSREGAQKEYVQHKMMEKAAQVWDLIKEEGYLYVCGDA
KGMARDVHRTLHTIVQEQEGVSSSEAEAIVKKLQTEGRYLRDVW

### Figure Description

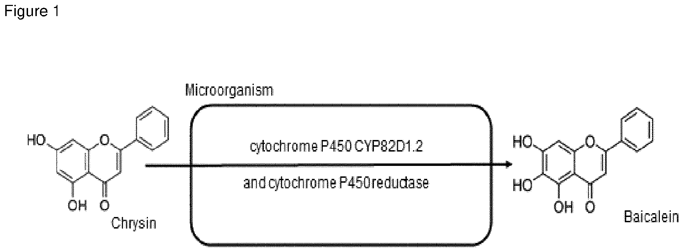

Figure 1: A genetically modified microorganism expressing cytochrome P450 CYP82D1.2 and cytochrome P450 reductase. Chrysin is used as a substrate and is converted to baicalein.

## Claims

1. A non-natural occurring microorganism which is able to biosynthetically produce baicalein (5,6,7-trihydroxyflavone), wherein said microorganism has been modified to comprise an enzymatic pathway for the biosynthetic production of baicalein.

2. A non-natural occurring microorganism which is able to biosynthetically produce baicalein according to claim 1, wherein the precursor for the biosynthetic production of baicalein is chrysin.

3. A non-natural occurring microorganism which is able to biosynthetically produce baicalein according to claims 1 and 2, wherein said enzymatic pathway comprises at least one cytochrome and at least one cytochrome reductase.

4. A non-natural occurring microorganism which is able to biosynthetically produce baicalein according to claims 1 to 3, wherein said microorganism has been modified to comprise cytochrome P450 and cytochrome P450 reductase.

5. A non-natural occurring microorganism which is able to biosynthetically produce baicalein according to claims 1 to 4, wherein said microorganism has been modified to comprise cytochrome P450 CYP82D1.2 and cytochrome P450 reductase.

6. A non-natural occurring microorganism which is able to biosynthetically produce baicalein according to claims 1 to 5, wherein the cytochrome P450 CYP82D1.2 is selected from SEQ ID_NO 1 or a protein sequence having at least 70% identity thereof, SEQ ID_NO 2 or a protein sequence having at least 70% identity thereof, and SEQ ID_NO 3 or a protein sequence having at least 70% identity thereof, and the cytochrome P450 reductase is selected from SEQ ID_NO 4 or a protein sequence having at least 70% identity thereof, SEQ ID_NO 5 or a protein sequence having at least 70% identity thereof, SEQ ID_NO 6 or a protein sequence having at least 70% identity thereof, and SEQ ID_NO 7 or a protein sequence having at least 70% identity thereof.

7. A non-natural occurring microorganism according to claims 1 to 6, wherein said microorganism is genetically modified with SEQ ID_NO 3 or a protein sequence having at least 70% identity thereof, and SEQ ID_NO 4 or a protein sequence having at least 70% identity thereof.

8. A non-natural occurring microorganism according to claims 1 to 7, wherein said microorganism is genetically modified with SEQ ID_NO 3 or a protein sequence having at least 70% identity thereof, and SEQ ID_NO 6 or a protein sequence having at least 70% identity thereof.

9. A non-natural occurring microorganism according to claims 1 to 8, wherein said microorganism is a bacterium.

10. A non-natural occurring microorganism according to claims 1 to 8, wherein said bacterium is from the *Escherichia* genus.

11. A non-natural occurring microorganism according to claim 10 wherein said bacteria is selected from the group comprising *Escherichia coli BLR (DE3), Escherichia coli Rosetta2 (DE3), Escherichia coli T7* and *Express Escherichia coli BL21 (DE3).*

12. A non-natural occurring microorganism according to claim 9 wherein said bacterium is from the *Zymomonas* genus, in particular from *Zymomonas mobilis.*

13. A non-natural occurring microorganism according to claim 9, wherein said bacterium is from the *Clostridium* genus, in particular from the *Clostridium autoethanogenum.*

14. A non-natural occurring microorganism according to claims 1 to 8, wherein said microorganism is a eukaryote.

15. A non-natural occurring microorganism according to claim 14, wherein said eukaryote is a *Saccharomyces,* in particular *Saccharomyces Cerevisiae.*

16. A method to produce baicalein, comprising a non-natural occurring microorganism according to claims 1-15, and in particular which has been modified to comprise cytochrome P450 CYP82D1.2 and cytochrome P450 reductase.

17. Use of a non-natural occurring microorganism according to claims 1-15 for biosynthetic production of baicalein from the precursor molecule chrysin, and in particular wherein said microorganism has been modified to comprise cytochrome P450 CYP82D1.2 and cytochrome P450 reductase.


SEQUENCE LISTING

<110> Synbionik Gmbh

<120> Non-naturally Occurring Microorganism for the Biosynthetic Production
of Baicalein.

<130> SynBaica

<160> 7

<170> BiSSAP 1.3.6

<210> 1
<211> 493
<212> PRT
<213> Scutellaria baicalensis

<220>
<223> Cytochrome P450 CYP82D1.2 (identified as CYPD1.2_whole) (protein
      ID: ASW21051.1)

<400> 1
Met Glu Leu Ser Ser Val Ile Tyr Gly Ala Ile Ala Leu Leu Ser Leu
1               5                   10                  15
Phe Tyr Cys Tyr Leu His Phe Ser Lys Pro Lys Lys Ser Ser Leu Asn
            20                  25                  30
Ala Pro Pro Glu Ala Gly Gly Ala Arg Phe Ile Thr Gly His Leu His
        35                  40                  45
Leu Met Asp Gly Arg Ser Ala Ser Asp Lys Leu Pro His Ile Asn Leu
    50                  55                  60
Gly Leu Leu Ala Asp Gln His Gly Pro Ile Phe Thr Ile Arg Leu Gly
65                  70                  75                  80
Val His Arg Ala Val Val Val Ser Ser Trp Glu Leu Ala Lys Glu Ile
                85                  90                  95
Phe Thr Thr His Asp Thr Ala Val Met Ala Arg Pro Arg Leu Ile Ala
            100                 105                 110
Asp Asp Tyr Leu Ser Tyr Asp Gly Ala Ser Leu Gly Phe Ser Pro Tyr
        115                 120                 125
Gly Pro Tyr Trp Arg Glu Ile Arg Lys Leu Val Thr Thr Glu Leu Tyr
    130                 135                 140
Lys Leu Trp Glu Glu Lys Lys Asp Gly Ser Gly Arg Val Leu Val Asp
145                 150                 155                 160
Met Lys Gln Trp Leu Gly Asn Leu Ser Leu Asn Leu Val Ser Arg Met
                165                 170                 175
Val Val Gly Lys Arg Phe Tyr Gly Gly Asp Asp Ser Glu Thr Thr Lys
            180                 185                 190
Arg Trp Arg Gly Val Met Arg Glu Phe Phe Gln Leu Ile Gly Gln Phe
        195                 200                 205
Ile Pro Gly Asp Gly Leu Pro Phe Leu Arg Trp Leu Asp Leu Gly Gly
    210                 215                 220
Phe Glu Lys Arg Thr Arg Asp Thr Ala Tyr Glu Leu Asp Lys Ile Ile
225                 230                 235                 240
Ala Met Trp Leu Ala Glu Tyr Arg Lys Arg Glu Tyr Ser Gly Asp Asp
                245                 250                 255
Lys Glu Gln Cys Phe Met Ala Leu Met Leu Ser Leu Val Gln Ala Asn
            260                 265                 270
Pro Thr Leu Gln Leu His Tyr Asp Ala Asp Thr Ile Ile Lys Ala Thr
        275                 280                 285
Cys Gln Val Leu Ile Ser Ala Ala Ser Asp Thr Thr Thr Val Ile Leu
    290                 295                 300
Ile Trp Val Ile Ser Leu Leu Leu Asn Asn Ala Asp Val Leu Lys Lys

305                 310                 315                 320
Val Gln Glu Glu Leu Asp Glu Gln Val Gly Arg Glu Arg Arg Val Glu
                325                 330                 335
Glu Ser Asp Ile Ser Asn Leu Pro Tyr Leu Gln Ala Val Val Lys Glu
            340                 345                 350
Thr Met Arg Leu Tyr Pro Pro Ala Pro Phe Ala Gly Val Arg Ala Phe
        355                 360                 365
Ser Glu Asp Cys Thr Val Gly Gly Tyr His Ile Gln Lys Gly Thr Phe
    370                 375                 380
Leu Ile Val Asn Leu Trp Lys Leu His Arg Asp Pro Arg Val Trp Ser
385                 390                 395                 400
Asp Asp Ala Leu Glu Phe Lys Pro Gln Arg Phe Phe Asp Lys Lys Val
                405                 410                 415
Glu Val Lys Gly Gln Asp Phe Glu Leu Met Pro Phe Gly Gly Gly Arg
            420                 425                 430
Arg Met Cys Pro Gly Ser Asn Leu Gly Met His Met Val His Phe Val
        435                 440                 445
Leu Ala Asn Ile Leu Gln Ala Phe Asp Ile Thr Thr Gly Ser Thr Val
    450                 455                 460
Asp Met Thr Glu Ser Val Gly Leu Thr Asn Met Lys Ala Thr Pro Leu
465                 470                 475                 480
Asp Ala Ile Leu Thr Pro Arg Leu Ser Pro Thr Leu Tyr
                485                 490

<210> 2
<211> 468
<212> PRT
<213> Scutellaria baicalensis


<220>
<223> CYPD1.2_TR

<400> 2
Pro Lys Lys Ser Ser Leu Asn Ala Pro Pro Glu Ala Gly Gly Ala Arg
1               5                   10                  15
Phe Ile Thr Gly His Leu His Leu Met Asp Gly Arg Ser Ala Ser Asp
            20                  25                  30
Lys Leu Pro His Ile Asn Leu Gly Leu Leu Ala Asp Gln His Gly Pro
        35                  40                  45
Ile Phe Thr Ile Arg Leu Gly Val His Arg Ala Val Val Val Ser Ser
    50                  55                  60
Trp Glu Leu Ala Lys Glu Ile Phe Thr Thr His Asp Thr Ala Val Met
65                  70                  75                  80
Ala Arg Pro Arg Leu Ile Ala Asp Asp Tyr Leu Ser Tyr Asp Gly Ala
                85                  90                  95
Ser Leu Gly Phe Ser Pro Tyr Gly Pro Tyr Trp Arg Glu Ile Arg Lys
            100                 105                 110
Leu Val Thr Thr Glu Leu Tyr Lys Leu Trp Glu Glu Lys Lys Asp Gly
        115                 120                 125
Ser Gly Arg Val Leu Val Asp Met Lys Gln Trp Leu Gly Asn Leu Ser
    130                 135                 140
Leu Asn Leu Val Ser Arg Met Val Val Gly Lys Arg Phe Tyr Gly Gly
145                 150                 155                 160
Asp Asp Ser Glu Thr Thr Lys Arg Trp Arg Gly Val Met Arg Glu Phe
                165                 170                 175
Phe Gln Leu Ile Gly Gln Phe Ile Pro Gly Asp Gly Leu Pro Phe Leu
            180                 185                 190
Arg Trp Leu Asp Leu Gly Gly Phe Glu Lys Arg Thr Arg Asp Thr Ala
        195                 200                 205
Tyr Glu Leu Asp Lys Ile Ile Ala Met Trp Leu Ala Glu Tyr Arg Lys
    210                 215                 220
Arg Glu Tyr Ser Gly Asp Asp Lys Glu Gln Cys Phe Met Ala Leu Met

225                 230                 235                 240
Leu Ser Leu Val Gln Ala Asn Pro Thr Leu Gln Leu His Tyr Asp Ala
                245                 250                 255
Asp Thr Ile Ile Lys Ala Thr Cys Gln Val Leu Ile Ser Ala Ala Ser
            260                 265                 270
Asp Thr Thr Thr Val Ile Leu Ile Trp Val Ile Ser Leu Leu Leu Asn
        275                 280                 285
Asn Ala Asp Val Leu Lys Lys Val Gln Glu Glu Leu Asp Glu Gln Val
    290                 295                 300
Gly Arg Glu Arg Arg Val Glu Glu Ser Asp Ile Ser Asn Leu Pro Tyr
305                 310                 315                 320
Leu Gln Ala Val Val Lys Glu Thr Met Arg Leu Tyr Pro Pro Ala Pro
                325                 330                 335
Phe Ala Gly Val Arg Ala Phe Ser Glu Asp Cys Thr Val Gly Gly Tyr
            340                 345                 350
His Ile Gln Lys Gly Thr Phe Leu Ile Val Asn Leu Trp Lys Leu His
        355                 360                 365
Arg Asp Pro Arg Val Trp Ser Asp Asp Ala Leu Glu Phe Lys Pro Gln
    370                 375                 380
Arg Phe Phe Asp Lys Lys Val Glu Val Lys Gly Gln Asp Phe Glu Leu
385                 390                 395                 400
Met Pro Phe Gly Gly Gly Arg Arg Met Cys Pro Gly Ser Asn Leu Gly
                405                 410                 415
Met His Met Val His Phe Val Leu Ala Asn Ile Leu Gln Ala Phe Asp
            420                 425                 430
Ile Thr Thr Gly Ser Thr Val Asp Met Thr Glu Ser Val Gly Leu Thr
        435                 440                 445
Asn Met Lys Ala Thr Pro Leu Asp Ala Ile Leu Thr Pro Arg Leu Ser
    450                 455                 460
Pro Thr Leu Tyr
465

<210> 3
<211> 493
<212> PRT
<213> Scutellaria baicalensis


<220>
<223> CYPD1.2_TR_SOL

<400> 3
Met Pro Lys Lys Ser Ser Leu Asn Ala Pro Pro Glu Ala Gly Gly Ala
1               5                   10                  15
Arg Phe Ile Thr Gly His Leu His Leu Met Asp Gly Arg Ser Ala Ser
            20                  25                  30
Asp Lys Leu Pro His Ile Asn Leu Gly Leu Leu Ala Asp Gln His Gly
        35                  40                  45
Pro Ile Phe Thr Ile Arg Leu Gly Val His Arg Ala Val Val Val Ser
    50                  55                  60
Ser Trp Glu Leu Ala Lys Glu Ile Phe Thr Thr His Asp Thr Ala Val
65                  70                  75                  80
Met Ala Arg Pro Arg Leu Ile Ala Asp Asp Tyr Leu Ser Tyr Asp Gly
                85                  90                  95
Ala Ser Leu Gly Phe Ser Pro Tyr Gly Pro Tyr Trp Arg Glu Ile Arg
            100                 105                 110
Lys Leu Val Thr Thr Glu Leu Leu Ser Ala Arg Arg Ile Glu Leu Gln
        115                 120                 125
Arg Ala Thr Arg Val Arg Glu Ile Thr Gln Phe Thr Gly Glu Leu Tyr
    130                 135                 140
Lys Leu Trp Glu Glu Lys Lys Asp Gly Ser Gly Arg Val Leu Val Asp
145                 150                 155                 160
Met Lys Gln Trp Leu Gly Asn Leu Ser Leu Asn Leu Val Ser Arg Met

                165                 170                 175
Val Val Gly Lys Arg Phe Tyr Gly Gly Asp Asp Ser Glu Thr Thr Lys
            180                 185                 190
Arg Trp Arg Gly Val Met Arg Glu Phe Phe Gln Leu Ile Gly Gln Phe
        195                 200                 205
Ile Pro Gly Asp Gly Leu Pro Phe Leu Arg Trp Leu Asp Leu Gly Gly
    210                 215                 220
Phe Glu Lys Arg Thr Arg Asp Thr Ala Tyr Glu Leu Asp Lys Ile Ile
225                 230                 235                 240
Ala Met Trp Leu Ala Glu Tyr Arg Lys Arg Glu Tyr Ser Gly Asp Asp
                245                 250                 255
Lys Glu Gln Cys Phe Met Ala Leu Met Leu Ser Leu Val Gln Ala Asn
            260                 265                 270
Pro Thr Leu Gln Leu His Tyr Asp Ala Asp Thr Ile Ile Lys Ala Thr
        275                 280                 285
Cys Gln Val Leu Ile Ser Ala Ala Ser Asp Thr Thr Thr Val Ile Leu
    290                 295                 300
Ile Trp Val Ile Ser Leu Leu Leu Asn Asn Ala Asp Val Leu Lys Lys
305                 310                 315                 320
Val Gln Glu Glu Leu Asp Glu Gln Val Gly Arg Glu Arg Arg Val Glu
                325                 330                 335
Glu Ser Asp Ile Ser Asn Leu Pro Tyr Leu Gln Ala Val Val Lys Glu
            340                 345                 350
Thr Met Arg Leu Tyr Pro Pro Ala Pro Phe Ala Gly Val Arg Ala Phe
        355                 360                 365
Ser Glu Asp Cys Thr Val Gly Gly Tyr His Ile Gln Lys Gly Thr Phe
    370                 375                 380
Leu Ile Val Asn Leu Trp Lys Leu His Arg Asp Pro Arg Val Trp Ser
385                 390                 395                 400
Asp Asp Ala Leu Glu Phe Lys Pro Gln Arg Phe Phe Asp Lys Lys Val
                405                 410                 415
Glu Val Lys Gly Gln Asp Phe Glu Leu Met Pro Phe Gly Gly Gly Arg
            420                 425                 430
Arg Met Cys Pro Gly Ser Asn Leu Gly Met His Met Val His Phe Val
        435                 440                 445
Leu Ala Asn Ile Leu Gln Ala Phe Asp Ile Thr Thr Gly Ser Thr Val
    450                 455                 460
Asp Met Thr Glu Ser Val Gly Leu Thr Asn Met Lys Ala Thr Pro Leu
465                 470                 475                 480
Asp Ala Ile Leu Thr Pro Arg Leu Ser Pro Thr Leu Tyr
                485                 490

<210> 4
<211> 711
<212> PRT
<213> Arabidopsis thaliana

<220>
<223> AtATR2

<400> 4
Met Ser Ser Ser Ser Ser Ser Ser Thr Ser Met Ile Asp Leu Met Ala
1               5                   10                  15
Ala Ile Ile Lys Gly Glu Pro Val Ile Val Ser Asp Pro Ala Asn Ala
            20                  25                  30
Ser Ala Tyr Glu Ser Val Ala Ala Glu Leu Ser Ser Met Leu Ile Glu
        35                  40                  45
Asn Arg Gln Phe Ala Met Ile Val Thr Thr Ser Ile Ala Val Leu Ile
    50                  55                  60
Gly Cys Ile Val Met Leu Val Trp Arg Arg Ser Gly Ser Gly Asn Ser
65                  70                  75                  80
Lys Arg Val Glu Pro Leu Lys Pro Leu Val Ile Lys Pro Arg Glu Glu

                85                  90                  95
Glu Ile Asp Asp Gly Arg Lys Lys Val Thr Ile Phe Phe Gly Thr Gln
            100                 105                 110
Thr Gly Thr Ala Glu Gly Phe Ala Lys Ala Leu Gly Glu Glu Ala Lys
        115                 120                 125
Ala Arg Tyr Glu Lys Thr Arg Phe Lys Ile Val Asp Leu Asp Asp Tyr
    130                 135                 140
Ala Ala Asp Asp Asp Glu Tyr Glu Glu Lys Leu Lys Lys Glu Asp Val
145                 150                 155                 160
Ala Phe Phe Phe Leu Ala Thr Tyr Gly Asp Gly Glu Pro Thr Asp Asn
                165                 170                 175
Ala Ala Arg Phe Tyr Lys Trp Phe Thr Glu Gly Asn Asp Arg Gly Glu
            180                 185                 190
Trp Leu Lys Asn Leu Lys Tyr Gly Val Phe Gly Leu Gly Asn Arg Gln
        195                 200                 205
Tyr Glu His Phe Asn Lys Val Ala Lys Val Val Asp Asp Ile Leu Val
    210                 215                 220
Glu Gln Gly Ala Gln Arg Leu Val Gln Val Gly Leu Gly Asp Asp Asp
225                 230                 235                 240
Gln Cys Ile Glu Asp Asp Phe Thr Ala Trp Arg Glu Ala Leu Trp Pro
                245                 250                 255
Glu Leu Asp Thr Ile Leu Arg Glu Glu Gly Asp Thr Ala Val Ala Thr
            260                 265                 270
Pro Tyr Thr Ala Ala Val Leu Glu Tyr Arg Val Ser Ile His Asp Ser
        275                 280                 285
Glu Asp Ala Lys Phe Asn Asp Ile Asn Met Ala Asn Gly Asn Gly Tyr
    290                 295                 300
Thr Val Phe Asp Ala Gln His Pro Tyr Lys Ala Asn Val Ala Val Lys
305                 310                 315                 320
Arg Glu Leu His Thr Pro Glu Ser Asp Arg Ser Cys Ile His Leu Glu
                325                 330                 335
Phe Asp Ile Ala Gly Ser Gly Leu Thr Tyr Glu Thr Gly Asp His Val
            340                 345                 350
Gly Val Leu Cys Asp Asn Leu Ser Glu Thr Val Asp Glu Ala Leu Arg
        355                 360                 365
Leu Leu Asp Met Ser Pro Asp Thr Tyr Phe Ser Leu His Ala Glu Lys
    370                 375                 380
Glu Asp Gly Thr Pro Ile Ser Ser Ser Leu Pro Pro Pro Phe Pro Pro
385                 390                 395                 400
Cys Asn Leu Arg Thr Ala Leu Thr Arg Tyr Ala Cys Leu Leu Ser Ser
                405                 410                 415
Pro Lys Lys Ser Ala Leu Val Ala Leu Ala Ala His Ala Ser Asp Pro
            420                 425                 430
Thr Glu Ala Glu Arg Leu Lys His Leu Ala Ser Pro Ala Gly Lys Asp
        435                 440                 445
Glu Tyr Ser Lys Trp Val Val Glu Ser Gln Arg Ser Leu Leu Glu Val
    450                 455                 460
Met Ala Glu Phe Pro Ser Ala Lys Pro Pro Leu Gly Val Phe Phe Ala
465                 470                 475                 480
Gly Val Ala Pro Arg Leu Gln Pro Arg Phe Tyr Ser Ile Ser Ser Ser
                485                 490                 495
Pro Lys Ile Ala Glu Thr Arg Ile His Val Thr Cys Ala Leu Val Tyr
            500                 505                 510
Glu Lys Met Pro Thr Gly Arg Ile His Lys Gly Val Cys Ser Thr Trp
        515                 520                 525
Met Lys Asn Ala Val Pro Tyr Glu Lys Ser Glu Asn Cys Ser Ser Ala
    530                 535                 540
Pro Ile Phe Val Arg Gln Ser Asn Phe Lys Leu Pro Ser Asp Ser Lys
545                 550                 555                 560
Val Pro Ile Ile Met Ile Gly Pro Gly Thr Gly Leu Ala Pro Phe Arg
                565                 570                 575
Gly Phe Leu Gln Glu Arg Leu Ala Leu Val Glu Ser Gly Val Glu Leu
            580                 585                 590

Gly Pro Ser Val Leu Phe Phe Gly Cys Arg Asn Arg Arg Met Asp Phe
        595                 600                 605
Ile Tyr Glu Glu Glu Leu Gln Arg Phe Val Glu Ser Gly Ala Leu Ala
    610                 615                 620
Glu Leu Ser Val Ala Phe Ser Arg Glu Gly Pro Thr Lys Glu Tyr Val
625                 630                 635                 640
Gln His Lys Met Met Asp Lys Ala Ser Asp Ile Trp Asn Met Ile Ser
                645                 650                 655
Gln Gly Ala Tyr Leu Tyr Val Cys Gly Asp Ala Lys Gly Met Ala Arg
            660                 665                 670
Asp Val His Arg Ser Leu His Thr Ile Ala Gln Glu Gln Gly Ser Met
        675                 680                 685
Asp Ser Thr Lys Ala Glu Gly Phe Val Lys Asn Leu Gln Thr Ser Gly
    690                 695                 700
Arg Tyr Leu Arg Asp Val Trp
705                 710

<210> 5
<211> 639
<212> PRT
<213> Arabidopsis thaliana

<220>
<223> AtATR2_TR

<400> 5
Arg Arg Ser Gly Ser Gly Asn Ser Lys Arg Val Glu Pro Leu Lys Pro
1               5                   10                  15
Leu Val Ile Lys Pro Arg Glu Glu Glu Ile Asp Asp Gly Arg Lys Lys
            20                  25                  30
Val Thr Ile Phe Phe Gly Thr Gln Thr Gly Thr Ala Glu Gly Phe Ala
        35                  40                  45
Lys Ala Leu Gly Glu Glu Ala Lys Ala Arg Tyr Glu Lys Thr Arg Phe
    50                  55                  60
Lys Ile Val Asp Leu Asp Asp Tyr Ala Ala Asp Asp Asp Glu Tyr Glu
65                  70                  75                  80
Glu Lys Leu Lys Lys Glu Asp Val Ala Phe Phe Phe Leu Ala Thr Tyr
                85                  90                  95
Gly Asp Gly Glu Pro Thr Asp Asn Ala Ala Arg Phe Tyr Lys Trp Phe
            100                 105                 110
Thr Glu Gly Asn Asp Arg Gly Glu Trp Leu Lys Asn Leu Lys Tyr Gly
        115                 120                 125
Val Phe Gly Leu Gly Asn Arg Gln Tyr Glu His Phe Asn Lys Val Ala
    130                 135                 140
Lys Val Val Asp Asp Ile Leu Val Glu Gln Gly Ala Gln Arg Leu Val
145                 150                 155                 160
Gln Val Gly Leu Gly Asp Asp Asp Gln Cys Ile Glu Asp Asp Phe Thr
                165                 170                 175
Ala Trp Arg Glu Ala Leu Trp Pro Glu Leu Asp Thr Ile Leu Arg Glu
            180                 185                 190
Glu Gly Asp Thr Ala Val Ala Thr Pro Tyr Thr Ala Ala Val Leu Glu
        195                 200                 205
Tyr Arg Val Ser Ile His Asp Ser Glu Asp Ala Lys Phe Asn Asp Ile
    210                 215                 220
Asn Met Ala Asn Gly Asn Gly Tyr Thr Val Phe Asp Ala Gln His Pro
225                 230                 235                 240
Tyr Lys Ala Asn Val Ala Val Lys Arg Glu Leu His Thr Pro Glu Ser
                245                 250                 255
Asp Arg Ser Cys Ile His Leu Glu Phe Asp Ile Ala Gly Ser Gly Leu
            260                 265                 270
Thr Tyr Glu Thr Gly Asp His Val Gly Val Leu Cys Asp Asn Leu Ser
        275                 280                 285

Glu Thr Val Asp Glu Ala Leu Arg Leu Leu Asp Met Ser Pro Asp Thr
    290                 295                 300
Tyr Phe Ser Leu His Ala Glu Lys Glu Asp Gly Thr Pro Ile Ser Ser
305                 310                 315                 320
Ser Leu Pro Pro Pro Phe Pro Pro Cys Asn Leu Arg Thr Ala Leu Thr
                325                 330                 335
Arg Tyr Ala Cys Leu Leu Ser Ser Pro Lys Lys Ser Ala Leu Val Ala
            340                 345                 350
Leu Ala Ala His Ala Ser Asp Pro Thr Glu Ala Glu Arg Leu Lys His
        355                 360                 365
Leu Ala Ser Pro Ala Gly Lys Asp Glu Tyr Ser Lys Trp Val Val Glu
    370                 375                 380
Ser Gln Arg Ser Leu Leu Glu Val Met Ala Glu Phe Pro Ser Ala Lys
385                 390                 395                 400
Pro Pro Leu Gly Val Phe Phe Ala Gly Val Ala Pro Arg Leu Gln Pro
                405                 410                 415
Arg Phe Tyr Ser Ile Ser Ser Ser Pro Lys Ile Ala Glu Thr Arg Ile
            420                 425                 430
His Val Thr Cys Ala Leu Val Tyr Glu Lys Met Pro Thr Gly Arg Ile
        435                 440                 445
His Lys Gly Val Cys Ser Thr Trp Met Lys Asn Ala Val Pro Tyr Glu
    450                 455                 460
Lys Ser Glu Asn Cys Ser Ser Ala Pro Ile Phe Val Arg Gln Ser Asn
465                 470                 475                 480
Phe Lys Leu Pro Ser Asp Ser Lys Val Pro Ile Ile Met Ile Gly Pro
                485                 490                 495
Gly Thr Gly Leu Ala Pro Phe Arg Gly Phe Leu Gln Glu Arg Leu Ala
            500                 505                 510
Leu Val Glu Ser Gly Val Glu Leu Gly Pro Ser Val Leu Phe Phe Gly
        515                 520                 525
Cys Arg Asn Arg Arg Met Asp Phe Ile Tyr Glu Glu Glu Leu Gln Arg
    530                 535                 540
Phe Val Glu Ser Gly Ala Leu Ala Glu Leu Ser Val Ala Phe Ser Arg
545                 550                 555                 560
Glu Gly Pro Thr Lys Glu Tyr Val Gln His Lys Met Met Asp Lys Ala
                565                 570                 575
Ser Asp Ile Trp Asn Met Ile Ser Gln Gly Ala Tyr Leu Tyr Val Cys
            580                 585                 590
Gly Asp Ala Lys Gly Met Ala Arg Asp Val His Arg Ser Leu His Thr
        595                 600                 605
Ile Ala Gln Glu Gln Gly Ser Met Asp Ser Thr Lys Ala Glu Gly Phe
    610                 615                 620
Val Lys Asn Leu Gln Thr Ser Gly Arg Tyr Leu Arg Asp Val Trp
625                 630                 635

<210> 6
<211> 692
<212> PRT
<213> Arabidopsis thaliana

<220>
<223> CPR1

<400> 6

<210> 7
<211> 666
<212> PRT
<213> Arabidopsis thaliana

<220>
<223> CPR1_TR

<400> 7
Val Val Leu Val Ile Ala Thr Thr Ser Leu Ala Leu Val Ala Gly Phe
1               5                   10                  15
Val Val Leu Leu Trp Lys Lys Thr Thr Ala Asp Arg Ser Gly Glu Leu
            20                  25                  30
Lys Pro Leu Met Ile Pro Lys Ser Leu Met Ala Lys Asp Glu Asp Asp
        35                  40                  45
Asp Leu Asp Leu Gly Ser Gly Lys Thr Arg Val Ser Ile Phe Phe Gly
    50                  55                  60
Thr Gln Thr Gly Thr Ala Glu Gly Phe Ala Lys Ala Leu Ser Glu Glu
65                  70                  75                  80
Ile Lys Ala Arg Tyr Glu Lys Ala Ala Val Lys Val Ile Asp Leu Asp
                85                  90                  95
Asp Tyr Ala Ala Asp Asp Asp Gln Tyr Glu Glu Lys Leu Lys Lys Glu
            100                 105                 110
Thr Leu Ala Phe Phe Cys Val Ala Thr Tyr Gly Asp Gly Glu Pro Thr
        115                 120                 125
Asp Asn Ala Ala Arg Phe Tyr Lys Trp Phe Thr Glu Glu Asn Glu Arg
    130                 135                 140
Asp Ile Lys Leu Gln Gln Leu Ala Tyr Gly Val Phe Ala Leu Gly Asn
145                 150                 155                 160
Arg Gln Tyr Glu His Phe Asn Lys Ile Gly Ile Val Leu Asp Glu Glu
                165                 170                 175
Leu Cys Lys Lys Gly Ala Lys Arg Leu Ile Glu Val Gly Leu Gly Asp
            180                 185                 190
Asp Asp Gln Ser Ile Glu Asp Asp Phe Asn Ala Trp Lys Glu Ser Leu
        195                 200                 205
Trp Ser Glu Leu Asp Lys Leu Leu Lys Asp Glu Asp Asp Lys Ser Val
    210                 215                 220
Ala Thr Pro Tyr Thr Ala Val Ile Pro Glu Tyr Arg Val Val Thr His
225                 230                 235                 240
Asp Pro Arg Phe Thr Thr Gln Lys Ser Met Glu Ser Asn Val Ala Asn
                245                 250                 255
Gly Asn Thr Thr Ile Asp Ile His His Pro Cys Arg Val Asp Val Ala
            260                 265                 270
Val Gln Lys Glu Leu His Thr His Glu Ser Asp Arg Ser Cys Ile His

        275                 280                 285
Leu Glu Phe Asp Ile Ser Arg Thr Gly Ile Thr Tyr Glu Thr Gly Asp
    290                 295                 300
His Val Gly Val Tyr Ala Glu Asn His Val Glu Ile Val Glu Glu Ala
305                 310                 315                 320
Gly Lys Leu Leu Gly His Ser Leu Asp Leu Val Phe Ser Ile His Ala
                325                 330                 335
Asp Lys Glu Asp Gly Ser Pro Leu Glu Ser Ala Val Pro Pro Pro Phe
            340                 345                 350
Pro Gly Pro Cys Thr Leu Gly Thr Gly Leu Ala Arg Tyr Ala Asp Leu
        355                 360                 365
Leu Asn Pro Pro Arg Lys Ser Ala Leu Val Ala Leu Ala Ala Tyr Ala
    370                 375                 380
Thr Glu Pro Ser Glu Ala Glu Lys Leu Lys His Leu Thr Ser Pro Asp
385                 390                 395                 400
Gly Lys Asp Glu Tyr Ser Gln Trp Ile Val Ala Ser Gln Arg Ser Leu
                405                 410                 415
Leu Glu Val Met Ala Ala Phe Pro Ser Ala Lys Pro Pro Leu Gly Val
            420                 425                 430
Phe Phe Ala Ala Ile Ala Pro Arg Leu Gln Pro Arg Tyr Tyr Ser Ile
        435                 440                 445
Ser Ser Ser Pro Arg Leu Ala Pro Ser Arg Val His Val Thr Ser Ala
    450                 455                 460
Leu Val Tyr Gly Pro Thr Pro Thr Gly Arg Ile His Lys Gly Val Cys
465                 470                 475                 480
Ser Thr Trp Met Lys Asn Ala Val Pro Ala Glu Lys Ser His Glu Cys
                485                 490                 495
Ser Gly Ala Pro Ile Phe Ile Arg Ala Ser Asn Phe Lys Leu Pro Ser
            500                 505                 510
Asn Pro Ser Thr Pro Ile Val Met Val Gly Pro Gly Thr Gly Leu Ala
        515                 520                 525
Pro Phe Arg Gly Phe Leu Gln Glu Arg Met Ala Leu Lys Glu Asp Gly
    530                 535                 540
Glu Glu Leu Gly Ser Ser Leu Leu Phe Phe Gly Cys Arg Asn Arg Gln
545                 550                 555                 560
Met Asp Phe Ile Tyr Glu Asp Glu Leu Asn Asn Phe Val Asp Gln Gly
                565                 570                 575
Val Ile Ser Glu Leu Ile Met Ala Phe Ser Arg Glu Gly Ala Gln Lys
            580                 585                 590
Glu Tyr Val Gln His Lys Met Met Glu Lys Ala Ala Gln Val Trp Asp
        595                 600                 605
Leu Ile Lys Glu Glu Gly Tyr Leu Tyr Val Cys Gly Asp Ala Lys Gly
    610                 615                 620
Met Ala Arg Asp Val His Arg Thr Leu His Thr Ile Val Gln Glu Gln
625                 630                 635                 640
Glu Gly Val Ser Ser Ser Glu Ala Glu Ala Ile Val Lys Lys Leu Gln
                645                 650                 655
Thr Glu Gly Arg Tyr Leu Arg Asp Val Trp
            660                 665